(19) Europäisches Patentamt
European Patent Office
Office européen des brevets

(11) EP 0 466 199 B1

(12) EUROPEAN PATENT SPECIFICATION

(45) Date of publication and mention of the grant of the patent:
**18.09.2002 Bulletin 2002/38**

(51) Int Cl.[7]: **C12N 15/12**, C07K 14/755

(21) Application number: **91113267.8**

(22) Date of filing: **11.01.1985**

(54) **Factor VIIIc encoding DNA sequences and related DNA constructs**

Für Faktor-VIIIc kodierende DNA-Sequenzen und verwandte DNA-Konstruktionen

ADN codant pour le facteur VIIIc et constructions d'ADN associées

(84) Designated Contracting States:
**AT BE CH DE FR GB IT LI LU NL SE**

(30) Priority: **12.01.1984 US 570062**
**26.10.1984 US 664919**

(43) Date of publication of application:
**15.01.1992 Bulletin 1992/03**

(60) Divisional application:
**00200860.5 / 1 006 182**

(62) Document number(s) of the earlier application(s) in accordance with Art. 76 EPC:
**85100223.8 / 0 150 735**

(73) Proprietors:
- **CHIRON CORPORATION**
  **Emeryville, California 94608 (US)**
- **NOVO NORDISK A/S**
  **2880 Bagsvaerd (DK)**

(72) Inventors:
- **Kuo,George**
  **San Francisco,California 94122 (US)**
- **Masiarz,Frank R.**
  **San Francisco,California 94131 (US)**
- **Truett,Martha**
  **Oakland,California 94611 (US)**
- **Valenzuela,Pablo**
  **Berkely CA 94705 (US)**
- **Rasmussen,Mirella Ezban**
  **DK-2100 Copenhagen (DK)**
- **Favaloro,Jennifer**
  **Carlton, 3035 Victoria (AU)**

(74) Representative: **Hallybone, Huw George et al**
**Carpmaels and Ransford,**
**43 Bloomsbury Square**
**London WC1A 2RA (GB)**

(56) References cited:
**EP-A- 0 160 457** **WO-A-84/04541**
**WO-A-85/01961**

- **BLOOD, vol. 61, no. 4, April 1983, pages 807-811; C.A. FULCHER ET AL: "Thrombin proteolysis of purified factor VIII procoagulant protein: Correlation of action with generation of specific polypeptide"**
- **PROCEEDINGS OF THE NATIONAL ACADEMY OF SCIENCES OF USA, vol. 79, March 1982, WASHINGTON US, pages 1648-1652; C.A. FULCHER ET AL: "Characterization of the human factor VIII procoagulant protein with a heterologous precipitating antibody"**
- **PROCEEDINGS OF THE NATIONAL ACADEMY OF SCIENCES OF USA, vol. 79, November 1982, WASHINGTON US, pages 6461-6464; K. KURACHI AND E.W. DAVIE: "Isolation and characterization of a cDNA coding for human factor IX"**
- **T. MANIATIS ET AL, "Molecular cloning: a laboratory manual", November 10, 1982, COLD SPRING HARBOUR LABORATORY, US, "Strategies for cDNA cloning", page 224 - page 227**
- **NATURE, vol. 303, June 9, 1983, LONDON GB, pages 474-475; A.L. BLOOM: "Benefits of cloning genes for clotting factors"**
- **NATURE, vol. 306, December 8, 1983, LONDON GB, page 528; J. MADDOX: "Factor VIII cloning"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- NATURE, vol. 312, November 22, 1984, LONDON GB, pages 330-337; W.I. WOOD ET AL: "Expression of active human factor VIII from recombinant DNA clones"
- NATURE, vol. 312, November 22, 1984, LONDON GB, pages 342-347; J.T. TOOLE ET AL: "Molecular cloning of a cDNA encoding human antihaemophilic factor"

## Description

It is the object of the present invention to provide a process for the synthesis of polypeptide fragments of human Factor VIIIC, particularly a fragment having a molecular weight of 77/80 kd, DNA sequences encoding portions of this polypeptide fragment, extrachromosomal elements comprising these DNA fragments, a complex probe for screening DNA sequences, corresponding DNA constructs, and monoclonal antibodies prepared with an immunogen comprising the above polypeptide fragments.

The above object is achieved according to the claims. The dependent claims relate to preferred embodiments.

In accordance with the above-defined object, the present invention particularly relates to a process for the synthesis of polypeptide fragments of human Factor VIIIC, particularly a fragment having a molecular weight of 77/80 kd, DNA sequences encoding portions of this polypeptide fragment, extrachromosomal elements comprising these DNA sequences, a complex probe for screening DNA sequences, and corresponding DNA constructs.

The parent patent EP 150 735 (application number 85 100223.8-2105) relates to an isolated protein composition comprising a calcium bridged complex of the 77/80 kd polypeptide fragment with a 92.5 kd fragment, exhibiting a coagulation activity similar to that of human Factor VIIIC.

Factor VIIIC is a plasma protein that participates in the intrinsic pathway of blood coagulation. It is absent or defective in individuals with the hereditary X chromosome-linked recessive bleeding disorder hemophilia A. Great difficulty has been encountered in isolating Factor VIIIC due to its extremely low concentration in plasma and the fact that it appears to be an intermediate or final degradation product of a larger protein precursor. Therefore, efforts to isolate Factor VIIIC have led to complex mixtures of significant heterogeneity and varying molecular weights.

One of the approaches which has found broad application to the production of physiologically active proteins involves the isolation of the protein of interest in purified form. The protein of interest provides invaluable aid in the development of a recombinant DNA capability for the production of the protein. By having the protein of interest, one may prepare monoclonal antibodies which are specific for the protein and can be used to establish the production of the protein in lysates, expression from messenger RNA in oocytes, or from a cDNA gene in unicellular microorganisms. In addition, by amino acid sequencing, one can develop probes, employing codons coding for the particular amino acid sequence, for hybridization to messenger RNA, chromosomal DNA or cDNA and, therefore, provide for the detection, isolation and expression of the relevant gene or message and the production of the desired product in high yield in one or more hosts.

US-A-4 361 509 and references cited therein describe purification of Factor VIIIC (see also Fulcher and Zimmerman, Proc. Natl. Acad. Sci. USA (1982) 79:1648-1652). Tuddenham et al., J. of Lab. Clinical Medicine (1979) 93, 40-53 describes purification of Factor VIIIC using polyclonal antibodies. Austen, British J. Hematology (1979) 43, 669-674 describes the use of aminohexyl-Sepharose for Factor VIIIC purification. Weinstein et al., Proc. Natl. Acad. Sci. USA (1981) 78, 5137-5141 describes a study of the effect of thrombin on Factor VIIIC. See also Kuo et al., Abstracts for IX International Congress of Thrombosis and Hemostasis, (Copenhagen; July, 1983).

It has further been known from C.A. Fulcher, J.R. Roberts and T.S. Zimmerman, Blood (1983) 61, No. 4, 807-811, that two polypeptide fragments of 79/80 kd and 92 kd can be isolated from the thrombin degradation product of human Factor VIIIC by SDS gel electrophoresis.

Methods and compositions are provided for production of human Factor VIIIC, precursors and subunits thereof, by expression in a microorganism or mammalian tissue culture cells. The method involves isolating pure Factor VIIIC, subunits and fragments thereof and determining their physiological relationship, particularly employing thrombin digestion. At least a portion of each of the related series of polypeptides is sequenced and the sequences employed for developing complex probes. Genomic DNA fragments are probed for homologous sequences and hybridizing fragments isolated and further manipulated to provide a DNA fragment encoding a complete subunit or fragment and/or used for isolating mature mRNA, from which ds cDNA may be obtained. The DNA sequence may then be further manipulated for insertion into an expression vector and the expression vector employed for introduction into a compatible host for expression of the polypeptide.

According to the invention, Human Factor VIIIC fragments and subunits may be provided in substantially pure form. In addition, methods and compositions are provided for the expression of Factor VIIIC subunits and fragments for producing Factor VIIIC as a precursor or in its active form or providing individual subunits for use in combination with naturally available subunits. The subunits and fragments have one or more biological properties associated with Factor VIIIC, such as epitopic sites, coagulation activity, and immunogenicity, so as to be used for producing antibodies which find use as reagents, particularly labeled reagents in immunoassays.

Human Factor VIIIC is a complex protein which can be isolated in substantially pure form exhibiting an apparent molecular weight of about 460kd. Upon electrophoresis under denaturing conditions, a large number of fragments result of varying molecular weights: 240, 160, 140, 115, 92.5, 80 and 77kd, the latter two being found to migrate as a doublet. Analysis of the fragments by chemical and protease cleavage including thrombin, employing antibodies to follow immunogenic relationships and cleavage patterns to follow structural relationships, demonstrates that the 92.5kd polypeptide is related to the 240, 160, 140 and 115 polypeptides while the 77/80 doublet may not have common identifiable characteristics with the other peptides, except for the 240kd polypeptide. It is further found that the 77/80kd doublet is converted by thrombin to a 67/70kd doublet, while the 92.5kd polypeptide present in purified Factor VIIIC material treated with thrombin is cleaved by thrombin, directly or indirectly, into two polypeptides of about 40 and 52.5kd. It is found that the electrophoretically isolated 77/80kd doublet polypeptides have their N-termini blocked, while the 67/70kd doublet polypeptides do not have their N-termini blocked.

It is further found that the locus for Factor VIIIC involves exons with large introns, where exons involve various domains associated with Factor VIIIC. Thus, individual exons can be isolated which involve specific polypeptides or fragments thereof involved with the Factor VIIIC complex. By identifying specific amino acid sequences involved with Factor VIIIC subunits and fragments thereof, one can selectively isolate the exons from genomic DNA and use the exons by themselves, in combination, or joined by synthetic DNA to provide for sequences encoding for and production of polypeptide subunits of Factor VIIIC or fragments thereof.

Conveniently, the Factor VIIIC genomic DNA sequences containing both exons and introns may be inserted into an expression vector appropriate for transcription and translation in mammalian cells to provide for both substantial quantities of properly spliced messenger RNA suitable for cDNA cloning and production of Factor VIIIC, subunits or fragments. In addition, the DNA sequences isolated from the genome can be used for hybridizing to natural messenger RNA encoding for Factor VIIIC. The messenger RNA may then be used to prepare ds cDNA for encoding the subunits of Factor VIIIC. The DNA sequences may be employed for expression by insertion into an appropriate expression vector having the necessary regulatory signals for transcription and translation. The Factor VIIIC gene expression vector (an expression vector carrying one or more genes encoding for all or a portion of Factor VIIIC, precursor, subunits or fragments thereof) may be introduced into a compatible host and the host grown for expression of Factor VIIIC. By appropriate choice of hosts, the Factor VIIIC DNA may be inserted downstream from a secretory leader and processing signals, so that the product will be secreted from the host and processed to provide for the complete polypeptide. As appropriate, the polypeptide may be further processed to introduce functionalities or substituents present on the naturally occurring polypeptide.

In the first stage of the subject invention, highly purified Factor VIIIC is obtained and characterized. Purified Factor VIIIC can be obtained from commercially available human anti-hemophilic factor (AHF), which is prepared from fresh, normal human plasma as a cryoprecipitate and represents about a 40-fold enrichment. The Factor VIIIC is further concentrated and purified by dissolving the anti-hemophilic factor into an appropriate buffer, e.g., saline imidazole-lysine-HCl, pH 7.4, followed by chromatography on an affinity column having either polyclonal or monoclonal antibodies to Factor VIIIC or Factor VIIIR. Conveniently, the antibodies are covalently bonded to a Sepharose support. Factor VIIIC may be eluted from the column employing a combination of a relatively high concentration of calcium ion with glycerol. The fractions obtained from the column may then be dialysed with an appropriate buffer, as described above, containing a low concentration of calcium ion and may then be further purified employing an aminohexyl-Sepharose column eluted with a high calcium or sodium chloride concentration buffer. Additional chromatographic steps, e.g., gelatin Sepharose, HPLC, ion exchange on dextran sulfate or Mono Q, affinity columns using lectins or antibodies to Factor VIIIC, provide additional purification. Particularly, the use of dextran sulfate removes trace contamination, e.g., fibrinogen, fibronectin, IgG, from the preparation, so as to leave a product substantially free of foreign proteins. Activity of the fractions from the columns may be monitored for either or both biological and antigenic activity using coagulation assay (commercially available kits) and antibodies specific for Factor VIIIC. Based on the concentration of Factor VIII in plasma, purifications of about 200,000-fold may be achieved by the above-described method.

Characterization of Factor VIIIC

Gel filtration indicates that Factor VIIIC behaves as a complex with an apparent molecular weight of about 460kd. Using SDS-gel electrophoresis (denaturing conditions) seven individual polypeptides can be isolated of differing molecular weight. The fragments as defined by their molecular weight are 240, 160, 140, 115, 92.5, 80 and 77kd. These fragments were characterized in the following ways.

The first study involved employing inhibitor antibodies isolated from hemophilic patients, the antibodies being designated as Z and E. Both antibodies reacted with the 77/80kd doublet. The E antibody reacted strongly with the 240kd polypeptide and weakly with several bands between the doublet and the 240kd polypeptide. The Z antibody also reacted weakly with the 240kd polypeptide.

In immunoprecipitation experiments, the E antibody precipitates the 77/80kd doublet as well as the high molecular weight species of 160, 140, 115 and 92.5kd, with the doublet among the stronger bands. Inclusion of EGTA results in the loss of the bands other than the doublet indicating that the 92.5kd species is associated with the 77kd and/or 80kd species in a complex mediated by a $Ca^{++}$ bridge.

In the next study, monoclonal antibodies were prepared, which both inhibit Factor VIIIC mediated coagulation activity and react with components of the complex: Class I reacting with the 77/80kd doublet and 240kd polypeptides;

Class II reacting with the 160, 140, 115 and 92.5kd polypeptides. Immunoprecipitation of thrombin-digested Factor VIIIC with Class I antibodies indicates that the 70/67kd doublet which results is derived from the 77/80kd doublet present in Factor VIIIC. The Class II monoclonals indicated that the 160, 140 and 115kd peptides are precursors of the 92.5kd peptide. A 40kd peptide cleavage product of 92.5kd peptide was also bound by the Class II antibodies. An ELISA assay using monoclonal antibodies in the presence and absence of EGTA confirms the $Ca^{++}$ bridge association between the 92.5kd and 77kd and/or 80kd components of the Factor VIIIC complex.

Both the human inhibitor and monoclonal antibodies may be used in immunosorbent column procedures to obtain Factor VIIIC or using EGTA, to resolve its constituent components, the 92.5kd and 77/80kd species.

The next study involved thrombin degradation of purified Factor VIIIC material at pH 6.8 or 7.4. Aliquots were assayed for coagulation activity and TCA precipitated for gel analysis. Coagulation activity was shown to increase with time and then decrease coincidently with an increase and decrease in the amount of the 92.5kd species. Thrombin treatment of the purified Factor VIIIC material for short periods of time (5-15min) enhances the amount of 92.5kd species, while the 77/80kd doublet is partially converted to a 67/70kd doublet. When long thrombin digestion times are employed, e.g., one hour, the 92.5kd protein is degraded and two new peptides of 40 and 52.5kd appear, with the 40kd peptide retaining immunogenic characteristics of the 92.5kd species. The 52.5kd peptide is shown to be a cleavage product by chemical and enzymatic degradation patterns and products analogous to the 92.5kd species.

In the next study individual Factor VIIIC subunits and precursors (e.g., 240, 77/80, 92.5kd species) were isolated by preparative SDS gel electrophoresis and a time course thrombin digestion of the isolated polypeptides was then performed. The 240kd fragment isolated from a preparative gel produced 160, 140, 115, and 92.5kd bands. The 80kd and 77kd fragments produced a 70kd and 67kd fragment, respectively.

A Factor VIIIC complex is derived containing the 77kd and/or 80kd species and 92.5kd polypeptide as a calcium-bridged complex in highly purified form. The purity of Factor VIIIC material (the complex and precursor species) is usually greater than 80%, often greater than 90%, and may be 98% or higher based on total protein, and that of the complex at least 20%, more usually 30% based on total protein, following the anti-Factor VIIIR immunosorbent and aminohexyl Sepharose columns. The use of additional chromatographic steps, e.g., dextran sulfate, increases the level of purity to at least 90% and usually greater for the Factor VIIIC material (complex plus precursor). The purity of Factor VIIIC components, 92.5kd species and the 77/80kd doublet, isolated by preparative SDS gel electrophoresis is usually at least 98%. As indicated above, the complex can be obtained using monoclonal antibodies specific for a member of the doublet, or for the 92.5kd polypeptide. The complex may then be separated from the antibody using a denaturing or chaotropic solvent, e.g., aqueous urea or thiocyanate, respectively.

<u>Preparation of Probes</u>

A partial amino acid sequence of the N-terminus of the 67 and 70kd polypeptides is as follows:

| | | | | | | | | | | |
|---|---|---|---|---|---|---|---|---|---|---|
| 1 | 2 | 3 | 4 | 5 | 6 | 7 | 8 | 9 | 10 | 11 |
| ? | Phe | Gln | Lys | Lys | Thr | Arg | His | Tyr | Phe | Ile |
| 12 | 13 | 14 | 15 | 16 | 17 | 18 | 19 | 20 | 21 | 22 |
| Ala | Ala | Val | Glu | Arg | Leu | Trp | Asp | Tyr | Gly | Met |

Based on this sequence, probes for the 67/70kd doublet (and thus the 77/80kd doublet from which it is derived) may be prepared having the following sequences:

```
Probe 1:  3' GTA ATA AAA TAA CGX CGX CA 5'        (X=G,C,A,T)
(non-coding)   G   G   G   G
                           T
```

```
Probe 2:  3' AAA GTT TTC TTC TGX TCT GT 5'
(non-coding)   G   C   T   T       C
                                  GCX
```

```
Probe 3:  5' GAA CGX TTA TGG GAT TAT GGX ATG 3'
(coding)       G AGA   G       C   C
                 G CTX
```

```
Probe 4:
(non-coding)
3' TCT GTA ATG AAA TAG CGA CGA CAC CTT TCT GAC ACC CTA ATG CCG TAC 5'
     C   G       G       G   G       C   C           G
```

The N-terminus amino acid sequence of the 52.5kd protein is substantially as follows:

| 1 | 2 | 3 | 4 | 5 | 6 | 7 | 8 | 9 | 10 | 11 | 12 | 13 |
|---|---|---|---|---|---|---|---|---|---|---|---|---|
| Ala | Thr | Arg | Arg | Tyr | Tyr | Leu | Gly | Ala | Val | Glu | Leu | Ser |

| 14 | 15 | 16 | 17 | 18 | 19 | 20 | 21 | 22 | 23 | 24 | 25 | 26 |
|---|---|---|---|---|---|---|---|---|---|---|---|---|
| Trp | Asp | Tyr | Met | Gln | Ser | Asp | ? | Gly | Glu | Leu | Pro | Val |

Based on this amino acid sequence, a probe for the 52.5kd protein may be prepared based on the coding strand as follows:

```
5'- GCA ACT AGA AGA TAT TAT TTG GGG GCA GTT GAA TTG TCA TGG GAT TAT -3'
         A   G   G               A   A       A       A   T
```

The amino acid sequences of three fragments of the 77/80kd proteins are as follows:

| 1 | 2 | 3 | 4 | 5 | 6 | 7 | 8 | 9 | 10 | 11 | 12 |
|---|---|---|---|---|---|---|---|---|---|---|---|
| Tyr | Ala | Ala | Thr | Ser | Gln | Val | Leu | Leu | Pro | Ser | Lys |

| 1 | 2 | 3 | 4 | 5 | 6 | 7 | 8 | 9 | 10 |
|---|---|---|---|---|---|---|---|---|---|
| Val | Thr | Gly | Val | Thr | Thr | Gln | Gly | Val | Lys |

| 1 | 2 | 3 | 4 | 5 | 6 | 7 | 8 | 9 | 10 | 11 | 12 |
|---|---|---|---|---|---|---|---|---|---|---|---|
| Met | Glu | Val | Leu | Gly | Cys | Glu | Ala | Gln | Asp | Leu | Tyr |

Based on these sequences respectively, probes may be prepared based on the non-coding strands as follows:

```
Probe 1:  3'-ATA  CGT  CGT  TGA  AGT  GTT  CAA  AAC  AAC  GG-5'
                              T    A         T    T    T

Probe 2:  3'-CAA  TGA  CCT  CAA  TGA  TGA  GTT  CCT  CAA  TTT-5'
               T    T         T    T    T              T

Probe 3:  3'-TAC  CTT  CAA  AAC  CCT  ACG  CTT  CGT  GTT  CTA  AAC  ATA-5'
                         T    T                                  T
```

Sequences of these peptides and preparation of the corresponding probes are described in detail in the Experimental section hereinafter.

Isolation of DNA

The above probes can be used for the detection and isolation of either genomic DNA or messenger RNA. Cloning genomic DNA involves cleavage of the genomic DNA with one or more restriction enzymes to obtain a partial digest and size selection of fragments of about 10-25kb. The restriction digests should be incomplete so that there will be overlapping fragments cloned. These fragments may be cloned in the appropriate vector to produce a "library" of clones in microorganisms, e.g., bacteria, such as E. coli. Various vectors may be employed, including plasmids or viruses, such as pBR322, lambda, charon 4A, EMBL-4, or the like.

The DNA is screened with the enzymatically radiolabeled probes described above and homologous sequences detected. Those sequences which hybridize with one or more of the probes may be recloned and again rehybridized one or more times.

One or more restriction enzymes different from the original restriction enzyme(s) employed may then be used to provide for smaller fragments, generally ranging from about 1-10kb, more usually from about 1-6kb. These fragments may then be subcloned and screened to identify positive fragments. The synthetic probes can then be used as primers for sequencing of the DNA fragments. Fragments which may be most conveniently sequenced are those which include a sequence complementary to one or more of the above-identified probes, where the homologous sequence is from about 5 bases and up to not more than about 500 bases from the 5'-terminus. Other fragments of interest include those at the termini of the original cloned fragment since these will be represented in other clones in the library and thus used to "walk" along the chromosome until the entire desired gene is recovered.

After sequencing the DNA fragment, based on the determined sequence, the fragment will be further manipulated. Based on the sequence, one can identify an open reading frame including the determined amino acid sequence. By determining restriction sites, one can further reduce the size of the fragment, without loss of coding sequences, although removal of a short sequence at the N-terminus is permissible, since this can be replaced by using appropriate adapters. Where restriction sites are not readily available at appropriate positions, the DNA fragment may be modified by Bal31 resection for varying times, the resulting fragments cloned, and the 5'-termini determined by various techniques. Conveniently, one can provide for a recognition site of a particular restriction enzyme by appropriate selection of the 3'-bases to which the resected fragment is joined. In this way, one can screen the resulting clones for the predetermined restriction site, which will indicate the presence of a fragment resected to the desired site.

Desirably exons or fragments thereof, usually of at least 50bp, more usually of at least about 100bp, even about 250bp or more, may be denatured and used as probes for mRNA from human cells, particularly cells producing mRNA for Factor VIIIC. By isolating hybridizing mRNA, the mRNA may be screened by translation in oocytes or a reticulocyte lysate and production of Factor VIIIC detected by antibodies to Factor VIIIC or coagulation activity based on binding to Factor VIIIC subunits. The mRNA may then be reverse transcribed, using, for example, AMV reverse transcriptase. Various methods can be used for converting ss cDNA to ds cDNA, using the reverse transcriptase or DNA polymerase I (Klenow fragment) to produce the second strand, followed by removal of the terminal loop, as appropriate, with a nuclease, e.g., $S_1$ nuclease. Where an incomplete copy is obtained, the messenger may be "walked" or primed cDNA synthesis may be used until the 5'-coding sequence of the mRNA has been copied and a DNA sequence encoding for the entire coding region of the mRNA is obtained.

Based on the above procedures, DNA sequences coding for the polypeptide precursor(s) to Factor VIIIC or major fragments thereof may be used for expression, or smaller fragments coding for specific subunits of Factor VIIIC, e.g., 92.5kd, 80kd or 77kd, may be employed.

For the precursor polypeptide, (proFactor VIIIC), the gene may be blunt-ended at one or both ends and inserted into an expression vector having complementary ends, or may be cleaved downstream from the 5'-coding terminus and joined to an adapter for appropriate insertion into the vector.

Fragments having the proper N-terminus, which may be at the coding sequence for the 70kd or 80kd polypeptide or may have a 5'-terminus downstream from the initial base, usually not more than about 30 bases downstream, more usually not more than about 20 bases downstream, may then be inserted into an appropriate vector using adapters, as appropriate.

Various vectors may be employed for providing extrachromosomal elements, depending upon the particular host, the manner of expression, whether constitutive or induced, the desired markers, whether secretion is desired, or the like. (By vector is intended an intact replication system.) Numerous vectors are presently available which provide for the transcriptional and translational regulatory signals recognized either by mammalian hosts, e.g., tissue culture cells or by prokaryotic and eukaryotic microorganism hosts, e.g., E. coli, B. subtilis, B. thermophilus, S. cerevisiae, or the like.

The vectors will have a replication system recognized by the host, although in some instances, integration of a construct having transcriptional and translational regulatory signals and the cistron of interest into the host genome may be desirable. In those situations, the construct will usually be flanked by sequences homologous to sequences in the host genome.

The expression vectors which are employed will have transcriptional and translational signals recognized by the host. The transcriptional signals will include the promoter and terminator, as well as auxiliary signals such as one or more enhancers. In addition, regulation of transcription may be provided, by including operators, activators, genes providing for repression, or the like. Other sequences involved with transcription include capping, polyadenylation, etc. For translation, depending upon the host, there may be a ribosomal binding site, an initiation codon, stop codons, or the like.

Conveniently, non-coding 5'- and 3'-flanking regions will be employed from genes native to the host, so that the signals recognized by the host will be present in appropriate relationship. These flanking regions can be joined to the gene encoding for the Factor VIIIC precursor, subunit or fragment thereof, so that the gene is in reading frame with the initiation codon and either carries its own stop codon or is inserted immediately upstream from one or more stop codons.

A vector will normally have one or more markers which provide for selection and allow for continued selective pressure during growth of the host. These markers may include prototrophy in an auxotrophic host, antibiotic resistance, toxin resistance, etc.

Where a secretory leader and processing signals are provided, it will usually be necessary to provide an adapter. By providing for an appropriate restriction site at the terminus of the DNA sequence encoding secretory leader and processing signals or upstream therefrom, one can synthesize an oligonucleotide adapter, usually of from about 10-50bp, which can be inserted between the secretory leader and processing signals or truncated portion thereof, and the gene of interest, which has a 5'-terminus at the initial codon of the gene or downstream thereof, so that the adapter restores all of the necessary missing bases and provides for the gene being in reading frame with the initiation codon of the leader sequence.

The resulting constructs which include the desired gene may then be introduced into a host, capable of growth in culture, in accordance with conventional methods, e.g., transformation, conjugation, transfection, or the like. The host may then be grown in an appropriate nutrient medium and the product isolated in accordance with conventional ways. Where the product is retained intracellularly, the cells will be harvested and lysed; where secreted, the product will be isolated from the nutrient medium. The product may be purified by chromatography, e.g., affinity chromatography, electrophoresis, extraction, HPLC, or the like.

For expression in a mammalian cell a mammalian virus may be employed as the vector, e.g., SV-40, papilloma virus, Maloney murine sarcoma virus, adenovirus, or the like. These viruses have been modified for use as expression vectors in mammalian cell cultures. An illustrative system employs COS cells bearing an integrated SV-40 genome and producing the large T antigen required for SV-40 replication (Gluzman, Cell (1981) 23:175). A fragment spanning the HpaI site at 0.76 on the SV-40 map to the BamHI site at 0.14 on the SV-40 map may be used as a vector. The recombinant plasmid obtained by joining the Factor VIIIC gene or portion thereof with the SV-40 vector may be used to transfect monkey CV-1 cells.

In accordance with the subject invention, purified subunits and fragments of Factor VIIIC may be obtained and used to enhance clotting capability for individuals requiring the particular subunit. The Factor VIIIC may also be used in therapy. In addition, the polypeptides prepared according to this invention can be used for the production of monoclonal antibodies to Factor VIIIC, its subunits and fragments. Also, the subunits and fragments may be used as reagents, which may be labeled and in combination with the antibodies, employed in diagnostic assays for the presence of one or more subunits or degradation fragments thereof in physiological fluids, e.g., blood or serum.

The following examples are offered by way of illustration and not by way of limitation.

Whenever used hereinafter Ab intends antibody and Ag antigen.

EXPERIMENTAL

I. Purification of Factor VIIIC

Human Factor VIIIC was isolated from commercial cryoprecipitate preparations by a) immunosorbent chromatography using a polyclonal anti VIIIR-Sepharose column by a method first described by E.G.D. Tuddenham, N.C. Trabold, J.A. Collins, and L.W. Hoyer, J. of Lab. Clinical Medicine (1979) 93:40; and b) a chromatographic separation on aminohexyl-substituted agarose as was originally described by D.E.G. Austen, British J. of Hematology (1979) 43: 669.

Details of the procedures are described below.

Goat anti-human Factor VIII Related Antigen (VIII:R) serum obtained from Atlantic Antibody (cat. no. 040-01), was treated with either a standard 0-50% ammonium sulfate cut followed by DEAE cellulose column chromatography, or a similar 0-33% cut without subsequent chromatography. These materials were then conjugated to CNBr-activated Sepharose CL2B or 4B, respectively, (Pharmacia, 17-0140-01 or 17-0430-01) and poured as a column (anti VIII: R-Sepharose column).

"HEMOFIL", a stable, dried preparation of antihemophilic factor (Factor VIII, AHF, AHG) in concentrated form prepared from fresh, normal human plasma, representing about a 40-fold enrichment for Factor VIIIC, was dissolved in the following buffer: 0.02M imidazole, 0.15M NaCl, 0.1M lysine-HCl, 0.02% $NaN_3$, pH 7.4.

After being dissolved, the Hemofil was applied to the above-described anti VIII:R-Sepharose column. Non-specifically bound protein was eluted with the above buffer modified to 0.5M NaCl. Next, Factor VIIIC was eluted with the above buffer containing 0.35M $CaCl_2$, with the addition of 10% glycerol which stabilizes the Factor VIIIC activity. Active fractions from the immunosorbent column were pooled and dialyzed against buffer (0.02M imidazole, 0.15M NaCl, 0.1M lysine-HCl, 0.025M $CaCl_2$, 0.02% $NaN_3$, 10% glycerol, pH 7.4). An aliquot of the dialyzed fractions, which contained 1,100 units of Factor VIIIC, was applied to an aminohexyl-Sepharose 4B column (1x6cm) equilibrated with dialysis buffer described above. Factor VIIIC activity was eluted with the same buffer containing either 0.35M $CaCl_2$ or 2M NaCl. The activity was found to be in a volume of 2ml with 500 units of Factor VIIIC per ml. Subsequent experiments carried out in the same manner provided a recovery of 25% off the anti VIII:R column and a recovery of approximately 90% off the aminohexyl column. Alternatively, pooled, dialysed material eluted from the immunosorbent column is first applied to a dextran sulfate (Pharmacia) column (1.5 x 6cm) equilibrated with the dialysis buffer above and eluted with the same buffer. Several minor contaminants, e.g., fibrinogen, fibronectin, IgG, are retained on the column while Factor VIIIC emerges in the flow-through which is collected and loaded on the aminohexyl-Sepharose column as before.

Both biological, i.e., clotting, and antigenic (cAg) activity were shown to be present in the purified Factor VIIIC, as demonstrated by the subsequent assays indicating a 5,000-fold purification over the 40-fold concentration in Hemofil. Using a standard commercially available three component kit from General Diagnostics, (APTT, Factor VIII deficient plasma, Verify Normal Citrate) a coagulation assay was carried out and indicated high levels of Factor VIIIC biological activity.

Antibodies employed were derived from inhibitor patients, one with a low titer (LZ) as coating ab and one with a high titer (HZ) as the labeled ab. The antibodies were used in two different types of assays. In an RIA assay, the HZ ab is labeled with $I^{125}$, while in an ELISA assay the HZ ab is coupled to horseradish peroxidase. Labeling with $^{125}I$ of antibody HZ for the RIA was performed in accordance with Hunter, W.M., in Radioimmunoassay, Weir, D.M., ed., Handbook of Experimental Immunology, 3rd ed., vol. 1, Blackwell Scientific Publications, Oxford, 1978. HRP-HZ conjugation was in accordance with Wilson and Nakane, in Immunofluorescence and Related Staining Techniques, Knapp et al., eds., Elsevier, North-Holland Biomedical Press, Amsterdam, 1978, pp. 215-224. LZ had an activity of 700 Bethesda Units/ml while HZ had an activity of 1,500 Bethesda Units/ml. Coating antibody (LZ) was diluted to 3.5µg/ml in 0.1M $NaHCO_3$, pH 9.8 (RIA) or 0.05M imidazole, 0.1M NaCl, 0.01% Thimerosal, 0.05% Tween 20, 5% BSA (ELISA) or for either method PBS-CMF (for 1 liter: 200mg KCl, 200mg $KH_2PO_4$, 8.0g NaCl, 1.15g anhydrous $Na_2HPO_4$, pH 7.4) and 1ml added to each tube (polystyrene) and incubated overnight at room temperature. This solution is removed by suction and the tubes washed 3x with 3-3.5ml 0.15M NaCl or PBS-CMF containing 0.05% Tween 20. Samples or standards (General Diagnostics, Verify Normal Citrate, catalog #34112) are diluted and added to the tubes to a total volume of 0.9ml per tube and incubated overnight at room temperature (dilutions were made in 0.02M Tris, 0.15M NaCl, 5% BSA, 0.05% Tween 20, 0.01% Thimerosal, pH 6.5 for RIA or 0.05M imidazole, 0.1M NaCl, 0.01% Thimerosal, 0.05% Tween 20, 5% BSA for ELISA or PBS-CMF for either method). Solutions were removed by suction and tubes washed as before. For RIA, 5 x $10^5$ cpm of $^{125}I$-labeled antibody to Factor VIIIC (HZ) in 600µl of RIA dilution buffer was added to each tube which was then incubated at 37°C for 16-18h; solutions were removed, the tubes washed as before and counted in a gamma counter. For ELISA, 0.9ml peroxidase conjugated anti-Factor VIIIC (HZ) was added to each tube whch was then incubated overnight at room temperature; solutions were removed and the tubes washed as before, then 0.9ml OPD solution (for 100ml: 0.73g citric acid, 1.19g disodium acid phosphate, 0.15g o-phenylenediamine, pH 5.0 with 250µl 10% $H_2O_2$ added immediately before use) added and incubated at room temperature for 30min in the dark. To stop this reaction, 0.5ml of 6N HCl (or 0.9ml 1M $H_2SO_4$) was added to each tube and the $OD_{492}$ read.

II. Structure of the Factor VIIIC Complex

A. Immunoprecipitation Experiments

Gel filtration experiments were carried out with an AcA 44 column on the Factor VIIIC purified material under the following conditions: 0.1% insulin (as carrier protein for stabilization), 0.25M $CaCl_2$, 0.01% Thiomerosal, 0.05M imidazole, pH 7.2. The Factor VIIIC coagulation and antigenic activities of the eluate were monitored. Two antigenic peaks were observed. One with Factor VIIIC coagulation activity behaved as a complex with an apparent molecular weight of about 460,000 under these conditions (native). The other peak (devoid of coagulation activity) eluted at an observed molecular weight slightly below 67,000.

When analyzed by standard analytical Laemmli SDS-gel electrophoresis (Laemmli, Nature (1970) 227: 680-685), various protein species of 240, 160, 140, 115, 92.5, 80 and 77kd were obtained. The relationship of these proteins to Factor VIIIC was determined by standard immunoprecipitation procedures. In the immunoprecipitation procedure, S. aureus protein A-Sepharose CL4B or polystyrene beads (1/8in, Precision Plastic Ball Co.) coated with affinity purified second antibody (goat anti-mouse IgG or anti-human IgG) were employed to separate antigen-Ab complexes from free $^{125}$I-labeled Factor VIIIC.

The proteins eluted from the affinity column were iodinated and then reacted with antibodies specific for Factor VIIIC. The antibodies were human inhibitor antibodies isolated from hemophiliac patients and referred to as anti-Factor VIIIC (Z) and (E) or inhibitor antibody (Z) and (E).

The results indicated that both antibodies reacted with the 77/80kd doublet. The "E" antibody also reacted strongly with the 240kd band and gave weak precipitation of several bands (160, 140, 115, 92.5kd) between the doublet and 240kd species. The "Z" antibody also precipitated the 92.5kd and 240kd proteins. The strong reaction of the "E" antibody with the 240kd species suggests that this species is a precursor of Factor VIIIC.

The antibody-column purified Factor VIIIC fraction was iodinated and reacted with the human inhibitor antibody in the presence and absence of EGTA (ethylene glycol bis(β-aminoethyl ether) N,N,N',N'-tetracetic acid). This allows for an investigation of the role of divalent cations, particularly $Ca^{++}$, in the association of the Factor VIIIC polypeptides. It was observed that the inhibitor antibody (E) precipitates the 77/80kd doublet, as well as higher molecular weight species of 160, 140, 115 and 92.5kd. The doublet is always among the stronger bands. (This immunoprecipitation experiment was done with the polystyrene beads. This procedure results in lower backgrounds and the labeled IgG in the Factor VIIIC preparation is not precipitated). Inclusion of EGTA results in the loss of the higher molecular weight bands (92.5-160kd) but has no effect on the amount of doublet precipitated. A similar experiment utilized Z antibody coupled to Sepharose as an immunosorbent: purified Factor VIIIC is applied to the column and after binding via 77/80kd, the 92.5kd polypeptide is selectively eluted with EDTA (ethylene diamine tetraacetic acid). The method is used preparatively to fractionate the 92.5kd species. This immunosorbent column or a similar one are prepared with polyclonal antibodies to Factor VIIIC. When eluted with chaotropic or denaturing solvents, e.g., thiocyanate solutions or aqueous urea, respectively, rather than EGTA, Factor VIIIC is further purified. These results suggest that the 92.5kd peptide may be associated noncovalently to the 77/80kd doublet via a $Ca^{++}$ bridge. Inhibitor antibody appears to interact directly only with the doublet. The higher molecular weight bands (the 115kd, 140kd, 160kd) are probably precursors of 92.5kd, as indicated by the ability of the monoclonal antibody directed against the 92.5kd polypeptide to cross-react with the 115kd, 140kd and 160kd polypeptides.

The relationship of various protein species from the affinity column was demonstrated by immunoprecipitation of iodinated, purified Factor VIIIC with monoclonal antibodies prepared according to the method of G. Kohler and C. Milstein (Eur. J. of Immunol. (1975) 6:511). Balb/c mice were immunized with liquid phase immunoadsorbed Factor VIIIC. Spleen cells ($10^8$) were fused with $10^7$ NSO or NSI mouse myeloma cells. The fusion products were plated into two 96-well microtiter trays. A spleen cell feeder layer was used at $10^4$ cells/well. Colonies were microscopically visible from the fifth day and the supernatants assayed every few days using an ELISA assay. The following layers were employed: 1st, Factor VIIIC eluted from hexyl-Sepharose 4B column, as described in Section I above; 2nd, hybridoma cell supernatant; 3rd, horseradish peroxidase (HRP)-labeled goat anti-mouse IgG; 4th, HRP-substrate.

Several classes of monoclonal antibodies were identified, two of which inhibited Factor VIIIC coagulation activity: Class I antibodies reacted with the 80/77kd doublet and 240kd polypeptides; and Class II antibodies reacted with proteins of 240, 160, 140, 115, 92.5kd. Immunoprecipitation of thrombin-digested Factor VIII with Class I monoclonal antibodies indicates that the 70/67kd doublet produced is derived from 77/80kd doublet (see below). Class III monoclonal antibodies indicate that the 160, 140 and 115kd peptides are precursors of 92.5kd peptide. The monoclonal antibodies of Class III further reacted with a 40kd peptide produced by thrombin digestion of the purified Factor VIIIC material.

An experiment similar to that described above, using EGTA to investigate the role of $Ca^{++}$ ion in the Factor VIIIC complex, was also performed, utilizing a monoclonal antibody based ELISA assay with the following layers: 1st, monospecific anti(mouse IgG); 2nd, Class III monoclonal antibody (anti-92.5kd); 3rd, purified Factor VIIIC material; 4th, HRP-human inhibitor antibody to 77/80kd. Addition of EGTA removed bound HRP activity present in the control without chelator. The fact that the Class I and III monoclonal antibodies directed to the 77/80kd doublet and 92.5kd proteins, respectively, are each inhibitory to Factor VIIIC coagulation activity implicates both as essential components of the Factor VIIIC complex.

B. Thrombin Activation of Factor VIIIC

Aminohexyl-concentrated, affinity-purified Factor VIIIC has been activated by thrombin (Boehringer, lot #1072302) using two different sets of pH conditions (6.8 and 7.4).

Aliquots were assayed for coagulation activity and, in addition, samples (about 2.5 units each) were TCA precipitated for gel analysis. In the first experiments, the VIIIC activity was initially 46 units/ml. This was diluted to a final concentration of 11.5 units/ml in Factor VIIIC buffer (20mM imidazole, pH 6.8, 150mM NaCl, 100mM lysine, 25mM $CaCl_2$ and 10% glycerol). The final concentration of thrombin was 0.12 unit/ml (about 1 unit of thrombin per 100 coagulation units of VIIIC). The results showed that the coagulation activity increases to about 180 units/ml then decreases to about 40 units/ml (essentially the starting value) coincidentally with a similar increase and decrease in the amount of 92.5kd species. Thus the 92.5kd species is implicated as part of the active Factor VIIIC complex.

Additional experiments with more concentrated Factor VIIIC preparations were carried out for the purpose of using thrombin activation in a preparative manner. To generate 92.5kd polypeptide, thrombin was added to the purified Factor VIIIC material (pH 7.4) at a ratio of about 1000-2000 coagulation units of Factor VIIIC to 1 unit of thrombin activity and allowed to react for only a short period of time (5-15min, depending on the Hemofil sample). The resulting product was then applied to a 7.5% preparative gel and .peptides separated by electrophoresis, the gel bands cut out and electroeluted.

When thrombin digestion is carried out for a short time, the amount of 92.5kd species can be doubled or tripled; at the same time, the 77/80kd doublet is only partially converted to 67/70kd species. To optimize conditions for isolation of the 67/70kd doublet, a longer time course (greater than lh) thrombin digestion is carried out. In this case, the 92.5kd species is further cleaved to produce smaller fragments. Two new peptides, 52.5kd and 40kd appear after thrombin treatment. The 40kd peptide reacts with the monoclonal antibody directed against the 92.5kd species and must therefore be a cleavage product. The 52.5kd peptide is also derived from the 92.5kd protein as demonstrated by a comparison of chemical and enzymatic cleavage patterns, i.e., both the 92.5kd and 52.5kd species when subjected to CNBr or endoproteinase lys C cleavage show a number of common fragments (by SDS-PAGE).

For endoproteinase lys C digestion, a weight ratio of lys C to protein of from about 1:1-100, usually 1:10, is used. In the subject digestion, 20pmoles (4.8μg) lys C was combined with 200pmoles (14μg) 70kd polypeptide in about 100μl 0.025M Tris-HCl, pH 7.7, 0.001M EDTA, 0.08% SDS and the mixture incubated at 37°C for 6h to overnight for complete digestion. Native polyacrylamide gels according to Orstein, Ann. N.Y. Acad. Sci. (1964) 121:321-349 were used for isolation of lys C digestion products.

C. Thrombin Digestion of Gel Isolated VIIIC-related Proteins

In order to confirm the precursor-product relationship of these peptides, a number of the bands were isolated by preparative SDS gel electrophoresis, electroeluted and subjected to thrombin digestion. The results were as follows:

1. The 240kd protein produced multiple bands including 160, 140, 115, 92.5kd but nothing smaller than 92.5kd, i.e., no 77/80kd or 67/70kd doublet. In addition, a time course for digestion was carried out with the 240kd fragment and analyzed for gel electrophoresis pattern, coagulation activity, and Factor VIIIC antigenic (Cag) activity. Gel results were the same as above and essentially no Cag or coagulation activity was recovered.
2. The 160kd and 92.5kd gel-isolated polypeptides do not appear to be substrates for thrombin after isolation from the gel.
3. Thrombin specifically cleaves gel isolated 77kd and 80kd species to produce new polypeptides of 67kd and 70kd, respectively. After thrombin treatment, monoclonal antibodies of Class I precipitate not only the 77/80kd doublet, but also the new 67 and 70kd species.

D. Amino Acid Sequence Analysis

Partial amino acid sequence information was obtained by standard procedures for the 67/70kd peptides, the 77/80kd peptides and the 52.5kd peptide isolated by preparative SDS electrophoresis. The electrophoretic analysis, together with the amino acid sequence results, indicated that the gel-isolated 77/80kd, 67/70kd, 92.5kd and 52.5kd polypeptides were obtained at >95%, usually 98%, purity. The gel isolated peptides were applied to a gas phase protein sequencer (Applied Biosystems). The PTH-amino acids were applied to an HPLC column (IBM cyano, 25cm) and the amino sequence determined from the resulting chromatograms.

The following sequence was determined for the 67/70kd doublet at its amino terminus (indicated with a bar in Appendix B):

| 1 | 2 | 3 | 4 | 5 | 6 | 7 | 8 | 9 | 10 | 11 |
|---|---|---|---|---|---|---|---|---|---|---|
| ? | Phe | Gln | Lys | Lys | Thr | Arg | His | Tyr | Phe | Ile |
| **12** | **13** | **14** | **15** | **16** | **17** | **18** | **19** | **20** | **21** | **22** |
| Ala | Ala | Val | Glu | Arg | Leu | Trp | Asp | Tyr | Gly | Met |

Using the information provided by the amino acid sequence of the N-terminal region of the 67/70kd protein the following oligonucleotide probes were synthesized to be used to screen human genomic libraries. The phosphoramidite method as described by M.S. Urdea et al., Proc. Natl. Acad. Sci. USA (1983) 80:7461-7465 was used:

```
Probe 1:  3' GTA ATA AAA TAA CGX CGX CA 5'        (X=G,C,A,T)
               G   G   G   G
                           T

Probe 2:  3' AAA GTT TTC TTC TGX TCT GT 5'
               G   C   T   T       C
                                 GCX

Probe 3:  5' GAA CGX TTA TGG GAT TAT GGX ATG 3'
               G AGA   G       C   C
                   G CTX

Probe 4:
3' TCT GTA ATG AAA TAG CGA CGA CAC CTT TCT GAC ACC CTA ATG CCG TAC 5'
     C   G       G       G   G       C   C           G
```

A scheme showing regions of amino acid sequence from which each probe is derived is shown below:

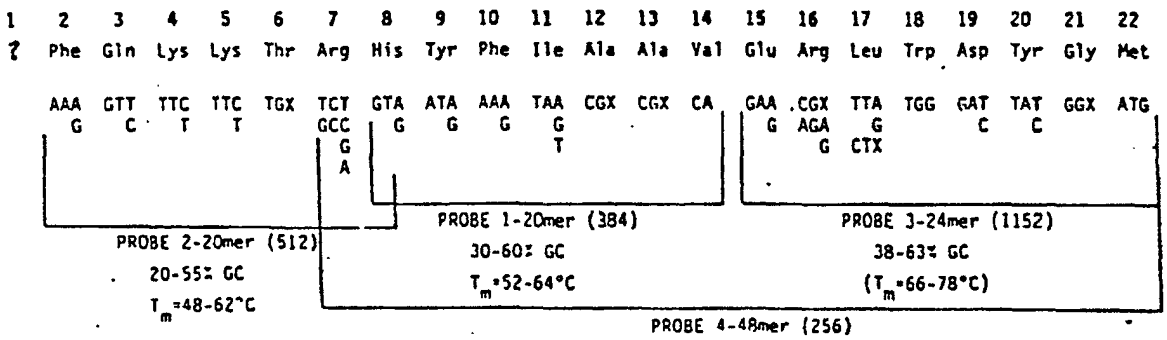


For the 52.5kd protein, which, as shown below, is derived from the 92.5kd protein, the following amino acid sequence at the N-terminus was determined:

| 1 | 2 | 3 | 4 | 5 | 6 | 7 | 8 | 9 | 10 | 11 | 12 | 13 |
|---|---|---|---|---|---|---|---|---|---|---|---|---|
| Ala | Thr | Arg | Arg | Tyr | Tyr | Leu | Gly | Ala | Val | Glu | Leu | Ser |
| **14** | **15** | **16** | **17** | **18** | **19** | **20** | **21** | **22** | **23** | **24** | **25** | **26** |
| Trp | Asp | Tyr | Met | Gln | Ser | Asp | ? | Gly | Glu | Leu | Pro | Val |

Based on the amino acid sequence for the 52.5kd peptide, a partially degenerate probe having the following nucleotide sequence (coding) was synthesized:

```
5'-GCA  ACT  AGA  AGA  TAT  TAT  TTG  GGG
        A    G    G              A    A

   GCA  GTT  GAA  TTG  TCA  TGG  GAT  TAT-3'
        A         A    T
```

This probe is useful for screening both genomic and cDNA libraries.

The amino acid sequences of two peptides obtained by digestion of the 77-80kd doublet with endoproteinase LysC (Boehringer-Mannheim) were determined. The digestion was performed as follows. The 77-80kd doublet was electrophoresed on an acrylamide protein gel and bands corresponding to the doublet were electroeluted. The separated material was purified and digested with endoproteinase LsyC, and the resulting peptides were separated by reverse phase HPLC. The fractions corresponding to peaks of absorbance at 280nm were sequenced using an automated sequencer (Applied Biosystems, Foster City, CA, Model A70A). The first sequence was as follows:

| 1 | 2 | 3 | 4 | 5 | 6 | 7 | 8 | 9 | 10 | 11 | 12 |
|---|---|---|---|---|---|---|---|---|---|---|---|
| Tyr | Ala | Ala | Thr | Ser | Gln | Val | Leu | Leu | Pro | Ser | Lys |

Based on this amino acid sequence, a partially degenerate probe having the following nucleotide sequence (non-coding) strand was synthesized:

```
3'-ATA  CGT  CGT  TGA  AGT  GTT  CAA  AAC  AAC  GG-5'
                   T    A         T    T    T
```

The second peptide had the following sequence:

| 1 | 2 | 3 | 4 | 5 | 6 | 7 | 8 | 9 | 10 |
|---|---|---|---|---|---|---|---|---|---|
| Val | Thr | Gly | Val | Thr | Thr | Gln | Gly | Val | Lys |

Based on this amino acid sequence, a partially degenerate probe having the following nucleotide sequence (non-coding) was prepared:

```
3'-CAA  TGA  CCT  CAA  TGA
     T    T         T    T

    TGA  GTT  CCT  CAA  TTT-5'
      T              T
```

The 77/80kd doublet was also digested with trypsin. The doublet material was purified as described for digestion with endoproteinase LysC, above. Lysines were blocked by citraconylation to allow digestion only at arginines. Citraconylation was performed by suspending the proteins in a denaturing buffer, reducing and carboxymethylating the suspended proteins, and treating with citraconic anhydride while maintaining a pH between 8.5 and 9.0. After citraconylation, the proteins were digested with trypsin, and the resulting peptides separated by reverse phase HPLC. The fractions corresponding to peaks of absorbance at 280nm were sequenced, as above. The sequence was as follows:

| 1 | 2 | 3 | 4 | 5 | 6 | 7 | 8 | 9 | 10 | 11 | 12 |
|---|---|---|---|---|---|---|---|---|---|---|---|
| Met | Gly | Val | Leu | Gly | Cys | Glu | Ala | Gln | Asp | Leu | Tyr |

Based on this amino acid sequence, a partially degenerate probe having the following nucleotide sequence (non-coding) was synthesized:

```
3'-TAC  CTT  CAA  AAC  CCT  ACG  CTT  CGT  GTT  CTA  AAC  ATA-5'
               T    T                                   T
```

When procedures to determine the N-terminus sequences of the 80 and 77Kd species were carried out, it was found that their amino termini were blocked. Therefore, the N-termini sequence was determined from material obtained by an alternative purification method which included immunoaffinity chromatography and ion exchange chromatography and precluded the use of preparative SDS-polyacrylamide gel electrophoresis. Purification of the 80/77Kd doublet involved application of Factor VIIIC concentrate to a monoclonal antibody column followed by chromatography on a mono S cation exchanger. This material had an unblocked N-termini, indicating that the blockage detected in gel purified 80/77Kd was an artifact resulting from the gel electrophoresis. The amino termini sequences determined for both 80 and 77Kd species is the following (indicated with a bar in Appendix B):

| 1 | 2 | 3 | 4 | 5 | 6 | 7 | 8 | 9 | 10 |
|---|---|---|---|---|---|---|---|---|---|
| Glu | Ile | Thr | ? | ? | ? | Leu | Gln | ? | Asp |
| 11 | 12 | 13 | 14 | 15 | 16 | 17 | 18 | 19 | 20 |
| Gln | Glu | Glu | Ile | Asp | Tyr | ? | Asp | Asp | Ile |

E. <u>Amino Acid Composition</u>

The amino acid compositions for the 77/80kd peptides were determined by standard methods to be as follows:

| Amino Acid | 80K | 77K |
|---|---|---|
| Asp | 58 | 54 |
| Glu | 74 | 76 |
| Cys | 12 | 14 |
| Ser | 47 | 44 |
| Gly | 51 | 46 |
| His | 8 | 12 |
| Arg | 32 | 29 |
| Thr | 35 | 29 |
| Ala | 35 | 33 |
| Pro | 33 | 30 |
| Tyr | 25 | 25 |
| Val | 46 | 44 |
| Met | 17 | 17 |
| Ile | 33 | 35 |
| Leu | 49 | 48 |
| Phe | 32 | 31 |
| Lys | 47 | 41 |
| Total No. Amino Acids | 634 | 608 |
| Calculated Molecular Weight: | 82K | 79K |

F. Preparation of Human 4X Genomic Library

Approximately 3mg of DNA were prepared from cell culture lysates of GM1416 cells (human lymphoblastoid cell line containing 4 copies of the X chromosome).

This DNA was partially digested with the restriction enzyme Sau3A, and the digested DNA (400-500µg) fractionated on 10%-40% sucrose gradients. Fractions in the size range 10-25 kilobases were pooled, dialyzed into Tris-EDTA and purified over Schleicher and Schuell Elutip-d sterile disposable columns. Aliquots of this DNA were ligated to EMBL-4 arms, obtained after digestion with BamHI and SalI and isolation on a gradient, and then packaged into bacteriophage lambda with an efficiency of $1x10^6$ pfu/µg of insert DNA. The vector used, EMBL-4, is a modified form of bacteriophage lambda (see Karn et al., Methods Enzymol. (1983) 101:3-19). The total library consisted of $5x10^6$ phage.

G. Plating and Screening of Human 4X Genomic Library

Bacteriophage were adsorbed to E. coli strain DP50 and 20 plates were plated at 50,000pfu per plate, (150x15mm size) to give $1x10^6$ pfu total. (Details of techniques for plates, top agar, adsorption and plating are found in Molecular Cloning, A Laboratory Manual, by T. Maniatis, E.F. Fritsch and J. Sambrook; Cold Spring Harbor Lab, New York, 1982.)

Nitrocellulose filters were applied to the surface of each plate containing phage plaques (so that molecules of unpackaged phage DNA are transferred to the filters) in duplicate, and hybridized with $^{32}$P-labeled 256-fold degenerate 48-mer probe DNA (probe #4). (Details of the nitrocellulose transfer technique are found in Maniatis et al., supra.) Pre-hybridization and hybridization were carried out in Wallace mix which contains in one liter: 310ml of distilled $H_2O$, 200ml 50% dextran sulfate, 180ml 1M Tris-HCl, pH 8.0, 225ml 4M NaCl, 20ml 0.25M EDTA, 50ml 100X Denhardt's solution, 5ml 100% NP-40 and 10ml 10% SDS.

The probe was labeled by enzymatic transfer of $^{32}PO_4$ from γ-$ATP^{32}$ to the 5' phosphate end of each probe DNA molecule, catalysed by T4 polynucleotide kinase. The hybridization conditions were as follows: 10ml hybridization mix/filter x 5000cpm of labeled probe #4/degeneracy/ml. Hybridization was carried out at 37°C overnight. Filters were washed in 6XSSC, 1mM EDTA at 50-55°C, air dried and used to expose X-ray film.

H. Characterization of Positive Clones

Twenty-three plaques giving positive signals for the first round of screening were replated, phage DNA transferred to nitrocellulose and hybridized with freshly labeled probe #4 (secondaries). Eleven plaques giving positive signals were replated, phage DNA transferred to nitrocellulose and hybridized with freshly labeled probe #4 (tertiaries).

Eight plaques giving positive signals were isolated and DNA prepared (100ml liquid cultures for each). The DNA corresponding to each of these 8 clones was digested with EcoRI (to release inserted human genomic DNA from the lambda vector DNA) and the resulting fragments separated by size using electrophoresis on a 0.8% agarose gel, denatured and transferred to nitrocellulose. This was done in quadruplicate and each filter hybridized with $^{32}P$-labeled probe #'s 1, 2, 3 or 4. The filters were used to expose to X-ray film and a single band of about 4.4kb in size was found to hybridize with all four probes for two clones. These two clones were identical except that one had more insert DNA than the other (clone designations are 23 D for the larger insert of 15.21kb and 11 for the smaller insert of approximately 13kb). The 4.4kb gel isolated EcoRI fragment was subcloned in vectors M13 and pUC-9 (a derivative of pBR322). DNA sequencing by the dideoxy technique on M13 DNA using the synthetic probe #3 and its reverse complement as primers was carried out.

The 4.4kb fragment was partially sequenced and has the following sequence encompassing the probe #4 sequence, indicated in parenthesis, and the partial amino acid sequence of the 67/70kd fragment originally determined, indicated in brackets.

```
        1                                       10
    val ser phe phe arg ala gln arg glu arg leu ser gly asn
GG  GTG TCC TTC TTC AGG GCT CAG AGG GAG CGA TTA AGT GGC AAC

                    20
glu ala asn arg pro gly lys leu pro phe leu arg val ala thr
GAA GCA AAC AGG CCT GGA AAA CTT CCC TTT CTG AGA GTA GCA ACA

30                                      40
glu thr leu gln arg leu pro pro ser tyr trp ile leu leu leu
GAA ACT CTG CAA AGA CTC CCT CCA AGC TAT TGG ATC CTC TTG CTT

                    50
gly ile pro leu trp tyr ser glu tyr gln lys lys ser gly lys
GGG ATA CCA CTA TGG TAC TCA GAG TAC CAA AAG AAG AGT GGA AAG

60                                      70
ser gln glu lys ser pro glu lys thr ala phe lys lys lys asp
TCC CAA GAG AAG TCA CCA GAA AAA ACA GCA TTT AAG AAA AAG GAT

                    80
thr ile leu ser leu asn ala cys glu ser asn his ala ile ala
ACC ATT TTG TCC CTG AAC GCT TGT GAA AGC AAT CAT GCA ATA GCA

90                                      100
ala ile asn glu gly gln asn lys pro glu ile glu val thr trp
GCA ATA AAT GAG GGA CAA AAT AAG CCC GAA ATA GAA GTC ACC TGG

                    110
ala lys gln asn arg thr glu arg leu cys ser gln asn pro pro
GCA AAG CAA AAT AGG ACT GAA AGG CTG TGC TCT CAA AAC CCA CCA

120                                     130
val leu lys arg his gln arg glu ile thr arg thr thr leu gln
GTC TTG AAA CGC CAT CAA CGG GAA ATA ACT CGT ACT ACT CTT CAG

                    140
ser asp gln glu glu ile asp tyr asp asp thr ile ser val glu
TCA GAT CAA GAG GAA ATT GAC TAT GAT GAT ACC ATA TCA GTT GAA

150                                     160
met lys lys glu asp phe asp ile tyr asp glu asp glu asn gln
ATG AAG AAG GAA GAT TTT GAC ATT TAT GAT GAG GAT GAA AAT CAG

                    170
ser pro arg ser[phe gln lys lys thr(arg his tyr phe ile ala
AGC CCC CGC AGC[TTT CAA AAG AAA ACA(CGA CAC TAT TTT ATT GCT

180                                     190
ala val glu arg leu trp asp tyr gly met)]ser ser ser pro his
GCA GTG GAG AGG CTC TGG GAT TAT GGG ATG)]AGT AGC TCC CCA CAT

                    200
val leu arg asn arg tyr glu cys ile gly tyr ser phe ala leu
GTT CTA AGA AAC AGG TAT GAA TGC ATT GGT TAT TCC TTT GCT CTG

210 211
leu leu OP
CTC TTG TGA CATTTGACTTTACCAGATGATGACACCAACC
```

This clone thus corresponds to the gene for the 77/80kd doublet protein, which, as it has been shown above, corresponds in part to the human Factor VIIIC complex.

Clone 23D was subcloned in phage M13 as EcoRI fragments, and the sequence corresponding to the inserted human DNA was determined. The complete 15.121kb sequence of clone 23D sequence is set forth in Appendix A, attached hereto. The subclone designations are given at the right hand margin of the sequence, and refer to the EcoRI-EcoRI fragment extending in the 3'-direction. An open reading frame of 3.110kb was found to exist from the 3'-end of the 70-3 fragment to the middle of the 4.4kb fragment. The open reading frame thus comprises at least part of the coding region for the 77/80kb doublet protein.

I. Comparison of Genomic DNA with cDNA

Three cDNA clones were obtained as follows. Clone C1 was obtained by screening a human liver cDNA library with a probe constructed from the 4.4kb EcoRI fragment of clone 23 D. Clone C2 was also obtained by screening a human liver cDNA library with the 4.4kb probe. Clone 2-11 was obtained by screening a human kidney cDNA library with a synthetic 45-mer probe based on the DNA sequence found at the 3'-end of the open reading frame of clone 23 D (nucleotides 9391 to 9435 in Appendix A). The probe comprised the non-coding strand of the following sequence:

```
                  Ser Pro Arg Ser Phe Gln Lys Lys
               5'-AGC CCC CGC AGC TTT CAA AAG AAA
Non-coding-    3'-TCG GGG GCG TCG AAA GTT TTG TTT
  (probe)

                  Thr Arg His Tyr Phe Ile Ala
                  ACA CGA CAC TAT TTT ATT GCT-3'
                  TGT GCT GTG ATA AAA TAA CGA-5'
```

The clones were sequenced and their locations relative to the genomic DNA of clone 23 D determined by comparing the sequences. Clone C1, which is 304kp in length, overlaps with the open reading frame from nucleotide 7773 to 8077, as numbered in Appendix A. Clone C2, which is 878bp in length, partially overlaps with the 3'-end of the open reading frame beginning at nucleotide 9538 and extending beyond nucleotide 9497, which is at the 3'-end of the open reading frame. Clone 2-11, which is 572 bp in length, also overlaps the 3'-end of the open reading frame beginning at nucleotide 9190 and extends beyond its termination. These findings thus confirm that the open reading frame is transcribed.

The coding information derived from the 4.4kb open reading frame may be combined with the additional coding information derived from clones 2-11 and C2 to prepare and extend sequence of 3.841kb corresponding to the total known coding sequence, as set forth in Appendix B. The regions corresponding to the C1, C2 and 2-11 probes are boxed.

To prepare Factor VIIIC, the DNA sequences from Clone 23 or Clone 11 are inserted into an SV-40 promoter as described by Laub et al., J. Virology (1983) 48:271, so as to be under the control of the SV-40 early promoter. The resulting recombinant plasmid may be transfected into COS cells (Guzman, supra.). Alternatively, the coding sequence can be inserted into a plasmid, e.g., pBR322, into which has been inserted the long terminal repeats of Maloney murine sarcoma virus, so that the Clone 23 or 11 sequences are under the transcriptional control of the viral regulatory system. The constructs may then be introduced into 3T3 mouse fibroblasts for efficient expression (see Perkins et al., Molecular and Cellular Biology, June 1983, Vol. 3, No. 6, p. 1123).

The above results demonstrate that genomic DNA has been isolated which codes for subunits of the Factor VIIIC complex. By use of this genomic DNA, the DNA may be further manipulated to provide for a sequence encoding for Factor VIIIC complex subunits. The DNA may then be used in an expression vector for production of the Factor VIIIC subunits, which may be used in a variety of ways, for example, as reagents in diagnostic assays, as therapeutic agents, for the production of monoclonal or polyclonal antibodies, which may then be used for the purification of Factor VIIIC complex or other purposes. The genomic DNA sequences may also be used for isolation of mRNA encoding proFactor VIIIC to provide for the precursor protein. This protein may then be administered for in vivo processing.

The subject techniques have provided a DNA sequence which includes the specific sequence at or adjacent to the 5'-terminus of the sequence coding for the 67/70kd doublet, which specific sequence is also present in the 77/80kd doublet, about 200 to 400, more exactly about 275 to 325 bases downstream from the 5'-terminus.

The bacteriophage λFVIII23D containing the 14.43kb EcoRI fragment was deposited at the A.T.C.C. on January 4, 1984 and given Accession No. 40094.

APPENDIX A

```
   1  GAATTCCCCGGATCTCTTATATTCCATTGACTTATTGGTCTATCTTTGTACCAGTAACAT  EcoRI #81
      CTTAAGGGGCCTAGAGAATATAAGGTAACTGAATAACCAGATAGAAACATGGTCATTGTA

      1 ecor1, 10 binI,

  61  ACTATCTTATAGTAAGTTTTAATATTTGTTAGTATAATTCCTCCAACTTTGCTCTCTTTG
      TGATAGAATATCATTCAAAATTATAAACAATCATATTAAGGAGGTTGAAACGAGAGAAAC

 121  ATTTTTATATAAATTTTAGAATCATCTTGTCAATTTTATCAATAAAAATATCTTCTGAGG
      TAAAAATATATTTAAAATCTTAGTAGAACAGTTAAAATAGTTATTTTTATAGAAGACTCC

      171 mbo11,

 181  TTTTGATAGGGATTACATTGATTCTGCAGATCAATTTGGGGGAGCATACCTCTTGACAAT
      AAAACTATCCCTAATGTAACTAAGACGTCTAGTTAAACCCCCTCGTATGGAGAACTGTTA

      204 pst1,

 241  ATTGAGTATTCCTGTCCATGAGCATGGTTTAGTTATTTATTTACATCTTTTAAATTTTCT
      TAACTCATAAGGACAGGTACTCGTACCAAATCAATAAATAAATGTAGAAAATTTAAAAGA

      256 bstXI, 289 aha111,

 301  CTTGGCAATGTTTTTGTAAAATTTTAGTGTACAGGTCTTGCACATTTTTGTAAAGTGTAT
      GAACCGTTACAAAAACATTTTAAAATCACATGTCCAGAACGTGTAAAAACATTTCACATA

 361  TCTTTGGTATTTAATGCTATTATAATATTTTTTATGGTATCAGTTTTTGTCAATTGTGTT
      AGAAACCATAAATTACGATAATATTATAAAAAATACCATAGTCAAAAACAGTTAACACAA

 421  TTTCTAGAAATATGTCCCTTTCATCTAAGTTGTTTAATTTACTTGAAGTAAGTTATTTGT
      AAAGATCTTTATACAGGGAAAGTAGATTCAACAAATTAAATGAACTTCATTCAATAAACA

      423 xba1,

 481  AATGTTCTTTTATCATCCTTATAATGTCTGTACTGATGTCTCCGTTTTCATACCTGATAT
      TTACAAGAAAATAGTAGGAATATTACAGACATGACTACAGAGGCAAAAGTATGGACTATA

 541  TGTTATTTATATTTTATCTATTTTAAGTTTATCGCCTGCACCCACTAACTCGTCATCTAG
      ACAATAAATATAAAATAGATAAAATTCAAATAGCGGACGTGGGTGATTGAGCAGTAGATC

 601  CATTAGGTATATCTCCCAATGATATCCCTCGCCCCTCCCCCTAGTGCACATGTACCCTAA
      GTAATCCATATAGAGGGTTACTATAGGGAGCGGGGAGGGGGATCACGTGTACATGGGATT

      621 ecor5,

 661  AACTTAAAGTATAATAAAAAAAAAAAAGTTTGTCGCCTTTGTTATAGGTTCATCAAATTTA
      TTGAATTTCATATTATTTTTTTTTTTTCAAACAGCGGAAACAATATCCAAGTAGTTTAAAT

 721  CTAATCCTACCATAGGATCAAATTTTTGCTTTGTTCATCAGGTCATGGAATTAAAAAAAT
      GATTAGGATGGTATCCTAGTTTAAAAACGAAACAAGTAGTCCAGTACCTTAATTTTTTTA

      735 binI,

 781  TTTTTTGTTTTGTTTATTTTCTATGTTGAATTTTTATTTTTATTTTGCTTTGCATTTCAT
      AAAAAACAAAACAAATAAAAGATACAACTTAAAAATAAAAATAAAACGAAACGTAAAGTA

 841  TTATTTCTGGTCTTTTGTTATTATTCCAATACTTTCTCTGGATTCAGTTTTCTTTTTCTT
      AATAAAGACCAGAAAACAATAATAAGGTTATGAAAGAGACCTAAGTCAAAAGAAAAAGAA

      897 mbo11,

 901  CTTGACTTCTTGAGATTAAAACTTGGTTCATTGATTTTCAACATTTCTTACTTTTTAAAA
      GAACTGAAGAACTCTAATTTTGAACCAAGTAACTAAAAGTTGTAAAGAATGAAAAATTTT

      954 aha111,

 961  GTTCATACAAAGCATAAATTTCCCTTTATATACTGCTTTAACCCCATATCACAAATTTTG
      CAAGTATGTTTCGTATTTAAAGGGAAATATATGACGAAATTGGGGTATAGTGTTTAAAAC

1021  ATGAGTCATATTTTTATTATCATTGAGTTTAAAATATTTTATAAATTTTACGTTATTTCT
      TACTCAGTATAAAAATAATAGTAACTCAAATTTTATAAAATATTTAAAATGCAATAAAGA

      1048 aha111,

1081  TGTTTTATTCATAGTTATTTTACAGTGTTTTACTTAATTTCCCAATATATGGCGATTTTC
      ACAAAATAAGTATCAATAAAATGTCACAAAATGAATTAAAGGGTTATATACCGCTAAAAG
```

1141 TGGCTCTCTCTCTGTTGCTGATTTATTTGTTTTACTGTGATCACATAACATACTCTATAT
ACCGAGAGAGAGACAACGACTAAATAAACAAAATGACACTAGTGTATTGTATGAGATATA

1178 bcl1,

1201 TATTTCAATCATTTGAAATTTGTTGAGACTTGCTATATGCCCAGTGAATAATTTAATTTG
ATAAAGTTAGTAAACTTTAAACAACTCTGAACGATATACGGGTCACTTATTAAATTAAAC

1261 ATTAATGGCCCACTGCATTTGAAACAAATATGTCTTTGCTGTTATTGGGTAGAGTGTCAT
TAATTACCGGGTGACGTAAACTTTGTTTATACAGAAACGACAATAACCCATCTCACAGTA

1321 ATATTTGCCTATTATGTCAAGTTGCTTTATCATGTTTTTAAAATCCTTATAAACTTCCTG
TATAAACGGATAATACAGTTCAACGAAATAGTACAAAAATTTTAGGAATATTTGAAGGAC

1357 aha111,

1381 ATATTTTATCTGTTTCTTACAGACATGGAGGAAACTGTGACATCTCTGTGATTGTTGAGT
TATAAAATAGACAAAGAATGTCTGTACCTCCTTTGACACTGTAGAGACACTAACAACTCA

1441 TTGTTATTTTGATTTTTGTATTTGTTAGTAAGAATGTTTCAAAATAAATGAGGCAATATA
AACAATAAAACTAAAAACATAAACAATCATTCTTACAAAGTTTTATTTACTCCGTTATAT

1501 AAATCAGGTGCTTACAACTTTAAGGTTGTATTCTTATTAAATCAATTCTTTCATCTTTGT
TTTAGTCCACGAATGTTGAAATTCCAACATAAGAATAATTTAGTTAAGAAAGTAGAAACA

1561 GAAATGTCCTTTTTGTCTGAAGTAGTACTTGTTGCCTTAAACGTCTGCATTGTCTAAAAT
CTTTACAGGAAAAACAGACTTCATCATGAACAACGGAATTTGCAGACGTAACAGATTTTA

1584 sca1,

1621 TTATACACCTGCACTATCATATTTATTTTGCTTAATGTTTGTGTGGTATATATTTCCCAT
AATATGTGGACGTGATAGTATAAATAAAACGAATTACAAACACACCATATATAAAGGGTA

1681 TCTTTTCTTTTCAACCTATCTCTATCCTTATATTTACAGTGTGTCTTTTTTAACATTTTC
AGAAAAGAAAAGTTGGATAGAGATAGGAATATAAATGTCACACAGAAAAAATTGTAAAAG

1741 TGTAGTGCAAGTCTGTTGGTTAATTAATTTTGTCAGTTTTTGCTTCTCTGAAAAGTCTTT
ACATCACGTTCAGACAACCAATTAATTAAAACAGTCAAAAACGAAGAGACTTTTCAGAAA

1801 ATTTCTGTTTTTGAATTATATCTTCACTGTGTATAGAATTCTGGGTTTGTGGTTGTTGTT EcoRI 61
TAAAGACAAAAACTTAATATAGAAGTGACACATATCTTAAGACCCAAACACCAACAACAA

1821 mbo11, 1836 ecor1,

1861 TTTGTTTTATTACATTTTGTTTTATTGGTTTTTCCCTCAGCACTTTAAAAATGCCGCTCC
AAACAAAATAATGTAAAACAAAATAACCAAAAAGGGAGTCGTGAAATTTTTACGGCGAGG

1904 aha111,

1921 ATTGTTTCTACTTGCATAGTTTTTCACAATAAGTTTGTTGTAATTCCCATTTGCATTCAA
TAACAAAGATGAACGTATCAAAAAGTGTTATTCAAACAACATTAAGGGTAAACGTAAGTT

1981 CTGTACATAATGTATCTTTTTAAAAAAATCTGGATGGCTTCAAGCTCTTTACTTTGATTT
GACATGTATTACATAGAAAAATTTTTTTAGACCTACCGAAGTTCGAGAAATGAAACTAAA

1999 aha111,

2041 TTGTGTGTGTGTGTGTGTGTGTGTGTGTGTGTGTGTGTGTGTTACTTTGGCATTCTCTAG
AACACACACACACACACACACACACACACACACACACACACAATGAAACCGTAAGAGATC

2101 GCTTATTCAATCTGTAATTTGGTATCATTAATTTTGAAAAATATTTATCATCTGTGTCTT
CGAATAAGTTAGACATTAAACCATAGTAATTAAAACTTTTTATAAATAGTAGACACAGAA

2157 mbo11,

2161 CAAATATTGCTTCTAATTCTTTCTCTTCTTCTGGTATTGCATTTACATATATTATACTGT
GTTTATAACGAAGATTAAGAAAGAGAAGAAGACCATAACGTAAATGTATATAATATGACA

2184 mbo11, 2187 mbo11,

2221 TCAATTTTGTCACACAGCTCTTGAATATGCTGTTCTAGTTTGTTTTCTAACTATTTTTCC
AGTTAAAACAGTGTGTCGAGAACTTATACGACAAGATCAAACAAAAGATTGATAAAAAGG

2281 TTCTAGTTGTTCAGTTTTGTTGACATTTATTGACCTATTTTTACTTCAGTAATTGTTTCC
AAGATCAACAAGTCAAAACAACTGTAAATAACTGGATAAAAATGAAGTCATTAACAAAGG

2341 TGGGTTGTTGAGTTAATTGATGAACTCATAGATGACATACAATTTATCTTTTTATTCTTG
ACCCAACAACTCAATTAACTACTTGAGTATCTACTGTATGTTAAATAGAAAAATAAGAAC

2401 TGTCACTTGTGCTTTTGATGTCACATCTAGGAAGGCTTTGTCTAGCCCAAGGCCACATTT
ACAGTGAACACGAAAACTACAGTGTAGATCCTTCCGAAACAGATCGGGTTCCGGTGTAAA

2461 ACTTCCATATTTTCTTCTAAGAGTTTGATAGTTGTAGCTGTTACATATATGTCTATGATC
TGAAGGTATAAAAGAAGATTCTCAAACTATCAACATCGACAATGTATATACAGATACTAG

2473 mbo11,

2521 TAGTTTGAATAAATTTTTGTGTATGGTGTGAAGAAGAGGTCTAACCTCTTTTTTATTTTT
ATCAAACTTATTTAAAAACACATACCACACTTCTTCTCCAGATTGGAGAAAAAATAAAAA

2550 mbo11, 2553 mbo11,

2581 TGCATGTAAACATCCAGTTGTCCCGGCACCATTTGTTGAAAATATGATTCTTTTCCCATT
ACGTACATTTGTAGGTCAACAGGGCCGTGGTAAACAACTTTTATACTAAGAAAAGGGTAA

2641 AAGTCATCTTGACACCCTTGTTGAAAATCAATTGACCATAAATGTGAGAGTTTATTTCTG
TTCAGTAGAACTGTGGGAACAACTTTTAGTTAACTGGTATTTACACTCTCAAATAAAGAC

2701 AACTCTCCATTCTGTTCCATTGACCCATACGTCTTCTATAAATTCCTTATAGCTAATTCT
TTGAGAGGTAAGACAAGGTAACTGGGTATGCAGAAGATATTTAAGGAATATCGATTAAGA

2732 mbo11,

2761 ACATTAGACTTTGGTTTTTCAGATGTGGTCATCTCATTAGATTATGGTGATATGTTTGGT
TGTAATCTGAAACCAAAAAGTCTACACCAGTAGAGTAATCTAATACCACTATACAAACCA

2821 TACAAGTTTTATCTTCTCCTTGGCAACATTTTTTTTATTACTGCTCTTCTTTTATTCGTT
ATGTTCAAAATAGAAGAGGAACCGTTGTAAAAAAAATAATGACGAGAAGAAAATAAGCAA

2832 mbo11, 2865 mbo11,

2881 TTTGTTGTTCCTTTCTCTTTTTGTGAGACAGGGTCTCACTCTGTTGCCCAGGGTGGAGTA
AAACAACAAGGAAAGAGAAAAACACTCTGTCCCAGAGTGAGACAACGGGTCCCACCTCAT

2907 tthIII1,

2941 AGTGGTGTGATCATGGCTCACTGCAGCCTCAACCTCCTGGGCTCAAGCAATCCTCCCACC
TCACCACACTAGTACCGAGTGACGTCGGAGTTGGAGGACCCGAGTTCGTTAGGAGGGTGG

2948 bcl1, 2961 pst1,

3001 TCAGCCTCCCAAGTAGTGGGACTACAGGCATGCACCATCATGCCAGGCTAACTTTTTTAT
AGTCGGAGGGTTCATCACCCTGATGTCCGTACGTGGTAGTACGGTCCGATTGAAAAAATA

3028 sph1,

3061 TTTTTGTAGAGACAGGGTCTTGCTTTGTTGCCCAGGCTGGTCTTGAACTCCAAGCTCAAG
AAAAACATCTCTGTCCCAGAACGAAACAACGGGTCCGACCAGAACTTGAGGTTCGAGTTC

3071 tthIII1,

3121 CAATCCTCCAACCTCAGCTTCCCAAAGTGCTGGGATTACCAGTATGAGCCACTGCACCTG
GTTAGGAGGTTGGAGTCGAAGGGTTTCACGACCCTAATGGTCATACTCGGTGACGTGGAC

3142 bstXI,

3181 GCCTTGTTGTTCCTTTCTTCCTTTTATCTTGTAGTATCTTGATCCTTTTTGCTTATTATT
CGGAACAACAAGGAAAGAAGGAAAATAGAACATCATAGAACTAGGAAAAACGAATAATAA

3196 mbo11,

3241 TATCATTGAGGTGAGTTTGTTCTAAATAAACTTTTCGCAGAAAGTTTATGTAGATATATT
ATAGTAACTCCACTCAAACAAGATTTATTTGAAAAGCGTCTTTCAAATACATCTATATAA

3301 AATATCAATGAGGCATTAGGAGTGAGTTTTAGATTAGCAGGAATTCTCTTATGAAAGGTA EcoRI #79
TTATAGTTACTCCGTAATCCTCACTCAAAATCTAATCGTCCTTAAGAGAATACTTTCCAT

3341 ecor1,

3361 TTGATTGTATGTGTGAGTTTTCAGCCTTTACTTCTCTCCAGCCCATCAAGATCAAAGCTG
AACTAACATACACACTCAAAAGTCGGAAATGAAGAGAGGTCGGGTAGTTCTAGTTTCGAC

3418 pst1,

3421 CAGAGTTGTTTACATTTCAGAATTTCTTTCCTCCCCTCCCCACCTTGCAAAACAGACTTA
GTCTCAACAAATGTAAAGTCTTAAAGAAAGGAGGGGAGGGGTGGAACGTTTTGTCTGAAT

3481 CAGTGCAAAAATGACTTGTCAATGTGATTCTTTCTTCTGGTCCCATCTTTCCTACCTTTT
GTCACGTTTTTACTGAACAGTTACACTAAGAAAGAAGACCAGGGTAGAAAGGATGGAAAA

3513 mbo11,

3541 GAGACTTGTGTTGAAAGGAGTCCAGTAAAACCACCGATGCTAGCTTGTATACCCTGATCC
CTCTGAACACAACTTTCCTCAGGTCATTTTGGTGGCTACGATCGAACATATGGGACTAGG

3601 CATTGTTATAATGAATGACTGACTGTTTTTCATATCAAAGTATAATACTTGCACCTTGAT
GTAACAATATTACTTACTGACTGACAAAAAGTATAGTTTCATATTATGAACGTGGAACTA

3661 AGAAGAAAGAAACAAGGCCAGGCACAGTGGTTCATGCCTGTAATCCCAGCACTTTGGGAG
TCTTCTTTCTTTGTTCCGGTCCGTGTCACCAAGTACGGACATTAGGGTCGTGAAACCCTC

3662 mbo11, 3706 bstXI,

3721 GCTGAAGTAGGAGGAATAATTAGACTTTGATATTAAAAATGGGGAAGATATGGCGGTATA
CGACTTCATCCTCCTTATTAATCTGAAACTATAATTTTTACCCCTTCTATACCGCCATAT

3764 mbo11,

3781 AGATATGAGGAAGTATGTCTTCTTGGACTTTTCTGAAATCTGCCAGCCATGAGCATCCAC
TCTATACTCCTTCATACAGAAGAACCTGAAAAGACTTTAGACGGTCGGTACTCGTAGGTG

3798 mbo11,

3841 TCTTTTTATCTTTTGATACCTGTTTTATATTAACTATACTTCTATTTTGTGGTTTCATGG
AGAAAAATAGAAAACTATGGACAAAATATAATTGATATGAAGATAAAACACCAAAGTACC

3901 CTTCAGATATCTTCCAGCTTAATTGTACTCTGTCTTTAATTATTTGCAGCAATTTCTGAA
GAAGTCTATAGAAGGTCGAATTAACATGAGACAGAAATTAATAAACGTCGTTAAAGACTT

3906 ecor5, 3910 mbo11,

3961 AGGAGCAAGAAGAAAGGCCAGCCCTTTCTTCACTACCTTAAAAACTGGTCATCTTTTGAA
TCCTCGTTCTTCTTTCCGGTCGGGAAAGAAGTGATGGAATTTTTGACCAGTAGAAAACTT

3969 mbo11, 3987 mbo11,

4021 ACCCCATGAAAGGCACAGTGGGTGACCATCTACATCAGGAGTGGGCAAACTATGGCCCTT
TGGGGTACTTTCCGTGTCACCCACTGGTAGATGTAGTCCTCACCCGTTTGATACCGGGAA

4041 bstE2,

4081 GGGTAGTCTGGTCTCTGTCCGTTTTTGTAAGGTTTTATTGGAACCCATACACACACAAAC
CCCATCAGACCAGAGACAGGCAAAAACATTCCAAAATAACCTTGGGTATGTGTGTGTTTG

4141 TGATCATCTCTTGTTATGTGTTAAGTTTTCTTATAATTATTCTTTTCTATTTCATCCAGC
ACTAGTAGAGAACAATACACAATTCAAAAGAATATTAATAAGAAAAGATAAAGTAGGTCG

4141 bcl1, 4197 pvu11,

4201 TGAATTACCATTTCACCTTGTGTATGCCCTTTCCAGTCTTAAATTATAGAAGCTATGTCT
ACTTAATGGTAAAGTGGAACACATACGGGAAAGGTCAGAATTTAATATCTTCGATACAGA

4261 TTGTGGTTTTAAATTATGGAAGCTGTGCCTTCTTGTGTTTTAGTATGGCAAGATATTTGT
AACACCAAAATTTAATACCTTCGACACGGAAGAACACAAAATCATACCGTTCTATAAACA

4268 aha111,

4321 TCTTACTTTTCCTCTAGAATCTGCAGTGGATTACTTTCAGAAGTAGGTATTGCCTCTAAG
AGAATGAAAAGGAGATCTTAGACGTCACCTAATGAAAGTCTTCATCCATAACGGAGATTC

4333 xba1, 4341 pst1,

4381 TTTCCTGACTTCTGTTTCCTTCTCCCTGTTCTGTAGTATATTTATAGATTGCATGTTTTC
AAAGGACTGAAGACAAAGGAAGAGGGACAAGACATCATATAAATATCTAACGTACAAAAG

4441 TCGTCCTTAAACAAGGTACAAATATGTTCTGATCAAGGTTTGCTAGCCGAGAGGATAAGT
AGCAGGAATTTGTTCCATGTTTATACAAGACTAGTTCCAAACGATCGGCTCTCCTATTCA

4470 bcl1,

4501 GGCTTGCTCTTAGCCTAACTCTCTGTCTACTTAATGGATGTCGAATTCCCTTCTCAACTC EcoRI #70-3
CCGAACGAGAATCGGATTGAGAGACAGATGAATTACCTACAGCTTAAGGGAAGAGTTGAG

4543 ecor1,

4561 AACAGCTAGTAACTAGTTCCAAGATCGGATAATAGCTTACAGAATTAATTAGTTCAGTGA

TTGTCGATCATTGATCAAGGTTCTAGCCTATTATCGAATGTCTTAATTAATCAAGTCACT

4621 AAGCCCTGGTTTGATCACAGAGGCAGGAGACTGATCTATATATTTGTAGTGCTGACTAGA

TTCGGGACCAAACTAGTGTCTCCGTCCTCTGACTAGATATATAAACATCACGACTGATCT

4632 bcl1,

4681 CCCCAACTTGGCCAGATAGCTTATACCTATGTCAGAAGTTGGAGGAGGAGATTGACAGCT

GGGGTTGAACCGGTCTATCGAATATGGATACAGTCTTCAACCTCCTCCTCTAACTGTCGA

4689 bal1, 4736 pvu11,

4741 GAGGTGTCTCCAAGGGAATTACAATTATTTTCATTTTCTTTGTGTCTGTAATTATGTCTC

CTCCACAGAGGTTCCCTTAATGTTAATAAAAGTAAAAGAAACACAGACATTAATACAGAG

4801 TTTTCTTACTTCAAATTTTGTATATGTGAAATTTCTTAATTTTTATTTGGTTATTTATTT

AAAAGAATGAAGTTTAAAACATATACACTTTAAAGAATTAAAAATAAACCAATAAATAAA

4861 TATTGACTTTTTCATCAAACAACCAACTCTAAGTTCTTGGATTGAAATTTATGAATTAAT

ATAACTGAAAAAGTAGTTTGTTGGTTGAGATTCAAGAACCTAACTTTAAATACTTAATTA

4921 TATAATGTTTTAAATTTACCATTAGTCACACATACAATACAAAAAGATATTTTCATTACA

ATATTACAAAATTTAAATGGTAATCAGTGTGTATGTTATGTTTTTCTATAAAAGTAATGT

4929 aha111,

4981 AAATTTTAACCCAAGCCATTAAAACTAAACTCCAGCTTGGTCACCATTCCTAATACCAGG

TTTAAAATTGGGTTCGGTAATTTTGATTTGAGGTCGAACCAGTGGTAAGGATTATGGTCC

5019 bstE2,

5041 CCCCTCTCAGAGGAGGGTAAATAAAAGTCCATTTTGGTAACTAGTTTGACGAGAATCTCT

GGGGAGAGTCTCCTCCCATTTATTTTCAGGTAAAACCATTGATCAAACTGCTCTTAGAGA

5101 AATTGTTTCCCTGTTTTTCTTATAATGTCTGCCTTTGAATTATCCTCTTTTTTTCAAGAG

TTAACAAAGGGACAAAAAGAATATTACAGACGGAAACTTAATAGGAGAAAAAAAGTTCTC

5161 TTCCATCATATAATGTATTCCTTTTTGCTTACAGATTGACCTCCTAGAGGCATGCATCCC

AAGGTAGTATATTACATAAGGAAAAACGAATGTCTAACTGGAGGATCTCCGTACGTAGGG

5210 sph1, 5212 ava3,

5221 CCTGCTCCAGTCTGAGCTGGTTCTCTCTAGGCCTGAGGCTCAGTTATCAACTTGGGACTT

GGACGAGGTCAGACTCGACCAAGAGAGATCCGGACTCCGAGTCAATAGTTGAACCCTGAA

5222 tthIII1, 5249 stu1, 5252 mstII,

5281 TTATTTACCCTCCCTCAGGATAGGTACCTGGTTCTTGGATACCAGGTCTTACTCTTGTTT

AATAAATGGGAGGGAGTCCTATCCATGGACCAAGAACCTATGGTCCAGAATGAGAACAAA

5293 mstII, 5303 kpn1,

5341 GAGATACTCCTTTATTTTGGTGGCAAACATTCCATAATCACTTCCTGATAATGGTGACAT

CTCTATGAGGAAATAAAACCACCGTTTGTAAGGTATTAGTGAAGGACTATTACCACTGTA

,01 TTGAGGTAGATTTTCTGAGTCTTTATATGTTAGAATATGTTTTTATTCTCCTTTGCCACT

AACTCCATCTAAAAGACTCAGAAATATACAATCTTATACAAAAATAAGAGGAAACGGTGA

61 TGATTGATAATTTGGCTGCACATAGAATGTAAGCATTCACAGGGCTTTTGGGAGCAGCTT

ACTAACTATTAAACCGACGTGTATCTTACATTCGTAAGTGTCCCGAAAACCCTCGTCGAA

5521 CCCTTGAGAAGAAGGTGGATGCAGTTAAGATCTTCCCTGAAAAGACTGTGTGAAATGACA

GGGAACTCTTCTTCCACCTACGTCAATTCTAGAAGGGACTTTTCTGACACACTTTACTGT

5528 mbo11, 5548 bgl11, 5551 mbo11,

5581 AGGCTATTGAGGTACCCAACGGGTAGCTTGGTAAAGGTGTATCTTGTCTCTTATGTTTAT

TCCGATAACTCCATGGGTTGCCCATCGAACCATTTCCACATAGAACAGAGAATACAAATA

5591 kpn1, 5639 ava3,

5641 GCATCTACTTTACCACCATGTAATTAGCCTATTATAACTTGTTGCATTCCTTCACTCAGC

CGTAGATGAAATGGTGGTACATTAATCGGATAATATTGAACAACGTAAGGAAGTGAGTCG

5701 TAATCTTGTTCATGGTAGAAATTAATATTGCTAGTACCATGGTAGAATTTTATATTAGCT

ATTAGAACAAGTACCATCTTTAATTATAACGATCATGGTACCATCTTAAAATATAATCGA

5737 nco1,

```
5761  GGATCCTAGACCTAAATGCTCCTTCCTTGTGCTTACATATTCTGCAAGTGGGTGACAGTG
      CCTAGGATCTGGATTTACGAGGAAGGAACACGAATGTATAAGACGTTCACCCACTGTCAC

      5761 bamh1 binI,

5821  GATGCTAGGTATTGTGTTATGTGATTTCAATGTATTATATGATGGGGTAGATACTATTAG
      CTACGATCCATAACACAATACACTAAAGTTACATAATATACTACCCCATCTATGATAATC

5881  CCTCATTTGAAAATTAGAAACCTATTGTTCAGAAAGTTTAAGTGACTTTCTCAAAGTCAC
      GGAGTAAACTTTTAATCTTTGGATAACAAGTCTTTCAAATTCACTGAAAGAGTTTCAGTG

5941  ACAGCTGGTAAATGGTAGAACTGGGATTTGAATCCAGGCAATCTGATGGATTTAAGAGGA
      TGTCGACCATTTACCATCTTGACCCTAAACTTAGGTCCGTTAGACTACCTAAATTCTCCT

      5942 pvu11,

6001  ACCTGTGCCACTATGTGATATAGCAATAACATCTAGCCAATATAAATACTTTCCTGAATT
      TGGACACGGTGATACACTATATCGTTATTGTAGATCGGTTATATTTATGAAAGGACTTAA

6061  AAATCACTGTCATATTCAGAGAGTTCTGTGTCACTATTAAGACCCTCCATTATTGTGTAT
      TTTAGTGACAGTATAAGTCTCTCAAGACACAGTGATAATTCTGGGAGGTAATAACACATA

      6063 ecopDXI,

6121  ATTAATTACATTTCCCTATTTTAATCCCAATATCTATTCATTTTTATCACAGTCCTTTCT
      TAATTAATGTAAAGGGATAAAATTAGGGTTATAGATAAGTAAAAATAGTGTCAGGAAAGA

6181  TATGGTCACAAACAGGCATAGTACAACAGCAGCAATGCAAAAACCACTTTTATATTACAT
      ATACCAGTGTTTGTCCGTATCATGTTGTCGTCGTTACGTTTTTGGTGAAAATATAATGTA

6241  GTTCCAGGGATTTAACCCAATGACCTGTGATATAATGATACTGACTAGTATTTTTTCATT
      CAAGGTCCCTAAATTGGGTTACTGGACACTATATTACTATGACTGATCATAAAAAAGTAA

6301  TATCTGGGAATGGGAGAGAACCTCTAACAGAACGTTTTAGAATCTGTGTTATGAGTAACC
      ATAGACCCTTACCCTCTCTTGGAGATTGTCTTGCAAAATCTTAGACACAATACTCATTGG

6361  AGAGTCTTAGTTCTTCCTCATCTCCAGGTCTATGGATTCTGGGGTGCCACAACTCAGACT
      TCTCAGAATCAAGAAGGAGTAGAGGTCCAGATACCTAAGACCCCACGGTGTTGAGTCTGA

      6372 mbo11, 6384 bstXI,

6421  TTCGGAAGCAGAGGCATGACCGCCTTACTGAAGGTTTCTAGTTGTGACAAGAACACTGGT
      AAGCCTTCGTCTCCGTACTGGCGGAATGACTTCCAAAGATCAACACTGTTCTTGTGACCA

6481  GATTATTACGAGGACAGTTATGAAGATATTTCAGCATACTTGCTGAGTAAAAACAATGCC
      CTAATAATGCTCCTGTCAATACTTCTATAAAGTCGTATGAACGACTCATTTTTGTTACGG

      6502 mbo11,

6541  ATTGAACCAAGAAGCTTCTCCCAGAATTCAAGACACCCTAGCACTAGGCAAAAGCAATTT  4.3kbEcoRI
      TAACTTGGTTCTTCGAAGAGGGTCTTAAGTTCTGTGGGATCGTGATCCGTTTTCGTTAAA

      6552 hind111, 6564 ecor1,

6601  AATGCCACCACAATTCCAGAAAATGACATAGAGAAGACTGACCCTTGGTTTGCACACAGA
      TTACGGTGGTGTTAAGGTCTTTTACTGTATCTCTTCTGACTGGGAACCAAACGTGTGTCT

      6633 mbo11,

6661  ACACCTATGCCTAAAATACAAAATGTCTCCTCTAGTGATTTGTTGATGCTCTTGCGACAG
      TGTGGATACGGATTTTATGTTTTACAGAGGAGATCACTAAACAACTACGAGAACGCTGTC

      6716 tthIII1,

6721  AGTCCTACTCCACATGGGCTATCCTTATCTGATCTCCAAGAAGCCAAATATGAGACTTTT
      TCAGGATGAGGTGTACCCGATAGGAATAGACTAGAGGTTCTTCGGTTTATACTCTGAAAA

6781  TCTGATGATCCATCACCTGGAGCAATAGACAGTAATAACAGCCTGTCTGAAATGACACAC
      AGACTACTAGGTAGTGGACCTCGTTATCTGTCATTATTGTCGGACAGACTTTACTGTGTG

6841  TTCAGGCCACAGCTCCATCACAGTGGGGACATGGTATTTACCCCTGAGTCAGGCCTCCAA
      AAGTCCGGTGTCGAGGTAGTGTCACCCCTGTACCATAAATGGGGACTCAGTCCGGAGGTT

      6855 bstXI, 6884 tthIII1, 6891 stu1,

6901  TTAAGATTAAATGAGAAACTGGGGACAACTGCAGCAACAGAGTTGAAGAAACTTGATTTC
      AATTCTAATTTACTCTTTGACCCCTGTTGACGTCGTTGTCTCAACTTCTTTGAACTAAAG

      6929 pst1, 6945 mbo11,
```

```
6961  AAAGTTTCTAGTACATCAAATAATCTGATTTCAACAATTCCATCAGACAATTTGGCAGCA
      TTTCAAAGATCATGTAGTTTATTAGACTAAAGTTGTTAAGGTAGTCTGTTAAACCGTCGT

7021  GGTACTGATAATACAAGTTCCTTAGGACCCCCAAGTATGCCAGTTCATTATGATAGTCAA
      CCATGACTATTATGTTCAAGGAATCCTGGGGGTTCATACGGTCAAGTAATACTATCAGTT

      7040 mstII,

7081  TTAGATACCACTCTATTTGGCAAAAAGTCATCTCCCCTTACTGAGTCTGGTGGACCTCTG
      AATCTATGGTGAGATAAACCGTTTTTCAGTAGAGGGGAATGACTCAGACCACCTGGAGAC

7141  AGCTTGAGTGAAGAAAATAATGATTCAAAGTTGTTAGAATCAGGTTTAATGAATAGCCAA
      TCGAACTCACTTCTTTTATTACTAAGTTTCAACAATCTTAGTCCAAATTACTTATCGGTT

      7150 mbo11,

7201  GAAAGTTCATGGGGAAAAAATGTATCGTCAACAGAGAGTGGTAGGTTATTTAAAGGGAAA
      CTTTCAAGTACCCCTTTTTTACATAGCAGTTGTCTCTCACCATCCAATAAATTTCCCTTT

      7249 aha111,

7261  AGAGCTCATGGACCTGCTTTGTTGACTAAAGATAATGCCTTATTCAAAGTTAGCATCTCT
      TCTCGAGTACCTGGACGAAACAACTGATTTCTATTACGGAATAAGTTTCAATCGTAGAGA

      7262 sac1,

7321  TTGTTAAAGACAAACAAAACTTCCAATAATTCAGCAACTAATAGAAAGACTCACATTGAT
      AACAATTTCTGTTTGTTTTGAAGGTTATTAAGTCGTTGATTATCTTTCTGAGTGTAACTA

7381  GGCCCATCATTATTAATTGAGAATAGTCCATCAGTCTGGCAAAATATATTAGAAAGTGAC
      CCGGGTAGTAATAATTAACTCTTATCAGGTAGTCAGACCGTTTTATATAATCTTTCACTG

      7408 bstXI,

7441  ACTGAGTTTAAAAAAGTGACACCTTTGATTCATGACAGAATGCTTATGGACAAAAATGCT
      TGACTCAAATTTTTTCACTGTGGAAACTAAGTACTGTCTTACGAATACCTGTTTTTACGA

      7447 aha111,

7501  ACAGCTTTGAGGCTAAATCATATGTCAAATAAAACTACTTCATCAAAAAACATGGAAATG
      TGTCGAAACTCCGATTTAGTATACAGTTTATTTTGATGAAGTAGTTTTTTGTACCTTTAC

      7519 nde1,

7561  GTCCAACAGAAAAAAGAGGGCCCCATTCCACCAGATGCACAAAATCCAGATATGTCGTTC
      CAGGTTGTCTTTTTTCTCCCGGGGTAAGGTGGTCTACGTGTTTTAGGTCTATACAGCAAG

      7578 apa1,

7621  TTTAAGATGCTATTCTTGCCAGAATCAGCAAGGTGGATACAAAGGACTCATGGAAAGAAC
      AAATTCTACGATAAGAACGGTCTTAGTCGTTCCACCTATGTTTCCTGAGTACCTTTCTTG

7681  TCTCTGAACTCTGGGCAAGGCCCCAGTCCAAAGCAATTAGTATCCTTAGGACCAGAAAAA
      AGAGACTTGAGACCCGTTCCGGGGTCAGGTTTCGTTAATCATAGGAATCCTGGTCTTTTT

      7724 mstII,

7741  TCTGTGGAAGGTCAGAATTTCTTGTCTGAGAAAAACAAAGTGGTAGTAGGAAAGGGTGAA
      AGACACCTTCCAGTCTTAAAGAACAGACTCTTTTTGTTTCACCATCATCCTTTCCCACTT

7801  TTTACAAAGGACGTAGGACTCAAAGAGATGGTTTTTCCAAGCAGCAGAAACCTATTTCTT
      AAATGTTTCCTGCATCCTGAGTTTCTCTACCAAAAAGGTTCGTCGTCTTTGGATAAAGAA

7861  ACTAACTTGGATAATTTACATGAAAATAATACACACAATCAAGAAAAAAAAATTCAGGAA
      TGATTGAACCTATTAAATGTACTTTTATTATGTGTGTTAGTTCTTTTTTTTTAAGTCCTT

      7918 mbo11,

7921  GAAATAGAAAAGAAGGAAACATTAATCCAAGAGAATGTAGTTTTGCCTCAGATACATACA
      CTTTATCTTTTCTTCCTTTGTAATTAGGTTCTCTTACATCAAAACGGAGTCTATGTATGT

7981  GTGACTGGCACTAAGAATTTCATGAAGAACCTTTTCTTACTGAGCACTAGGCAAAATGTA
      CACTGACCGTGATTCTTAAAGTACTTCTTGGAAAAGAATGACTCGTGATCCGTTTTACAT

      8004 mbo11, 8007 xmn1,

8041  GAAGGTTCATATGACGGGGCATATGCTCCAGTACTTCAAGATTTTAGGTCATTAAATGAT
      CTTCCAAGTATACTGCCCCGTATACGAGGTCATGAAGTTCTAAAATCCAGTAATTTACTA

      8048 nde1, 8060 nde1, 8070 sca1,
```

```
8101  TCAACAAATAGAACAAAGAAACACACAGCTCATTTCTCAAAAAAAGGGGAGGAAGAAAAC
      AGTTGTTTATCTTGTTTCTTTGTGTGTCGAGTAAAGAGTTTTTTTCCCCTCCTTCTTTTG

      8152 mbo11,

8161  TTGGAAGGCTTGGGAAATCAAACCAAGCAAATTGTAGAGAAATATGCATGCACCACAAGG
      AACCTTCCGAACCCTTTAGTTTGGTTCGTTTAACATCTCTTTATACGTACGTGGTGTTCC

      8204 ava3, 8206 sph1, 8220 ecor5,

8221  ATATCTCCTAATACAAGCCAGCAGAATTTTGTCACGCAACGTAGTAAGAGAGCTTTGAAA
      TATAGAGGATTATGTTCGGTCGTCTTAAAACAGTGCGTTGCATCATTCTCTCGAAACTTT

      8277 xmn1,

8281  CAATTCAGACTCCCACTAGAAGAAACAGAACTTGAAAAAAGGATAATTGTGGATGACACC
      GTTAAGTCTGAGGGTGATCTTCTTTGTCTTGAACTTTTTTCCTATTAACACCTACTGTGG

      8299 mbo11,

8341  TCAACCCAGTGGTCCAAAAACATGAAACATTTGACCCCGAGCACCCTCACACAGATAGAC
      AGTTGGGTCACCAGGTTTTTGTACTTTGTAAACTGGGGCTCGTGGGAGTGTGTCTATCTG

      8376 ava1,

8401  TACAATGAGAAGGAGAAAGGGGCCATTACTCAGTCTCCCTTATCAGATTGCCTTACGAGG
      ATGTTACTCTTCCTCTTTCCCCGGTAATGAGTCAGAGGGAATAGTCTAACGGAATGCTCC

8461  AGTCATAGCATCCCTCAAGCAAATAGATCTCCATTACCCATTGCAAAGGTATCATCATTT
      TCAGTATCGTAGGGAGTTCGTTTATCTAGAGGTAATGGGTAACGTTTCCATAGTAGTAAA

      8485 bgl11,

8521  CCATCTATTAGACCTATATATCTGACCAGGGTCCTATTCCAAGACAACTCTTCTCATCTT
      GGTAGATAATCTGGATATATAGACTGGTCCCAGGATAAGGTTCTGTTGAGAAGAGTAGAA

      8569 mbo11, 8577 mbo11,

8581  CCAGCAGCATCTTATAGAAAGAAAGATTCTGGGGTCCAAGAAAGCAGTCATTTCTTACAA
      GGTCGTCGTAGAATATCTTTCTTTCTAAGACCCCAGGTTCTTTCGTCAGTAAAGAATGTT

8641  GGAGCCAAAAAAAATAACCTTTCTTTAGCCATTCTAACCTTGGAGATGACTGGTGATCAA
      CCTCGGTTTTTTTTATTGGAAAGAAATCGGTAAGATTGGAACCTCTACTGACCACTAGTT

      8694 bcl1,

8701  AGAGAGGTTGGCTCCCTGGGGACAAGTGCCACAAATTCAGTCACATACAAGAAAGTTGAG
      TCTCTCCAACCGAGGGACCCCTGTTCACGGTGTTTAAGTCAGTGTATGTTCTTTCAACTC

8761  AACACTGTTCTCCCGAAACCAGACTTGCCCAAAACATCTGGCAAAGTTGAATTGCTTCCA
      TTGTGACAAGAGGGCTTTGGTCTGAACGGGTTTTGTAGACCGTTTCAACTTAACGAAGGT

      8809 xmn1,

8821  AAAGTTCACATTTATCAGAAGGACCTATTCCCTACGGAAACTAGCAATGGGTCTCCTGGC
      TTTCAAGTGTAAATAGTCTTCCTGGATAAGGGATGCCTTTGATCGTTACCCAGAGGACCG

      8877 bal1,

8881  CATCTGGATCTCGTGGAAGGGAGCCTTCTTCAGGGAACAGAGGGAGCGATTAAGTGGAAT
      GTAGACCTAGAGCACCTTCCCTCGGAAGAAGTCCCTTGTCTCCCTCGCTAATTCACCTTA

      8886 binI, 8907 mbo11,

8941  GAAGCAAACAGACCTGGAAAAGTTCCCTTTCTGAGAGTAGCAACAGAAAGCTCTGCAAAG
      CTTCGTTTGTCTGGACCTTTTCAAGGGAAAGACTCTCATCGTTGTCTTTCGAGACGTTTC

9001  ACTCCCTCCAAGCTATTGGATCCTCTTGCTTGGGATAACCACTATGGTACTCAGATACCA
      TGAGGGAGGTTCGATAACCTAGGAGAACGAACCCTATTGGTGATACCATGAGTCTATGGT

      9008 bstXI, 9018 bamh1 binI,

9061  AAAGAAGAGTGGAAATCCCAAGAGAAGTCACCAGAAAAAACAGCTTTTAAGAAAAAGGAT
      TTTCTTCTCACCTTTAGGGTTCTCTTCAGTGGTCTTTTTTGTCGAAAATTCTTTTTCCTA

      9064 mbo11,

9121  ACCATTTTGTCCCTGAACGCTTGTGAAAGCAATCATGCAATAGCAGCAATAAATGAGGGA
      TGGTAAAACAGGGACTTGCGAACACTTTCGTTAGTACGTTATCGTCGTTATTTACTCCCT
```

```
9181  CAAAATAAGCCCGAAATAGAAGTCACCTGGGCAAAGCAAGGTAGGACTGAAAGGCTGTGC
      GTTTTATTCGGGCTTTATCTTCAGTGGACCCGTTTCGTTCCATCCTGACTTTCCGACACG

9241  TCTCAAAACCCACCAGTCTTGAAACGCCATCAACGGGAAATAACTCGTACTACTCTTCAG
      AGAGTTTTGGGTGGTCAGAACTTTGCGGTAGTTGCCCTTTATTGAGCATGATGAGAAGTC

      9294 mbo11,

9301  TCAGATCAAGAGGAAATTGACTATGATGATACCATATCAGTTGAAATGAAGAAGGAAGAT
      AGTCTAGTTCTCCTTTAACTGATACTACTATGGTATAGTCAACTTTACTTCTTCCTTCTA

      9348 mbo11, 9355 mbo11,

9361  TTTGACATTTATGATGAGGATGAAAATCAGAGCCCCCGCAGCTTTCAAAAGAAAACACGA
      AAACTGTAAATACTACTCCTACTTTTAGTCTCGGGGGCGTCGAAAGTTTTCTTTTGTGCT

9421  CACTATTTTATTGCTGCAGTGGAGAGGCTCTGGGATTATGGGATGAGTAGCTCCCCACAT
      GTGATAAAATAACGACGTCACCTCTCCGAGACCCTAATACCCTACTCATCGAGGGGTGTA

      9434 pst1,

9481  GTTCTAAGAAACAGGTATGAATGCATTGGTTATTCCTTTGCTCTGCTCTTGTGACATTTG
      CAAGATTCTTTGTCCATACTTACGTAACCAATAAGGAAACGAGACGAGAACACTGTAAAC

      9501 ava3,

9541  ACTTTACCAGATGATGACACCAACAATGAGAGTTAGAGGATGAAAAATCAAGGTGTCTTG
      TGAAATGGTCTACTACTGTGGTTGTTACTCTCAATCTCCTACTTTTTAGTTCCACAGAAC

9601  AAACTCTTACTCTGGTGAGATAGAGAGAAGAGAGGTTTACAAGAGGGTAGAGGAATCAGG
      TTTGAGAATGAGACCACTCTATCTCTCTTCTCTCCAAATGTTCTCCCATCTCCTTAGTCC

      9627 mbo11,

9661  GACAAGAAGGAGCAATGGGGAGCAGGAAGAAAGTCAACCTCACCTATCAACCTTATGAGC
      CTGTTCTTCCTCGTTACCCCTCGTCCTTCTTTCAGTTGGAGTGGATAGTTGGAATACTCG

      9686 mbo11, 9720 pst1,

9721  TGCAGGGGAAATCTTAGTCTCATCAGAGAACTTTAAGCAAGGCAGAAGGAAGAAGCAAGT
      ACGTCCCCTTTAGAATCAGAGTAGTCTCTTGAAATTCGTTCCGTCTTCCTTCTTCGTTCA

      9769 mbo11,

9781  TCGTGTTAAAGAGGTTAAGGACTTGGGGAGGATTGGTTTTCATGATACTGGGGATCCAGA
      AGCACAATTTCTCCAATTCCTGAACCCCTCCTAACCAAAAGTACTATGACCCCTAGGTCT

      9832 bamh1 binI,

  '41  GGGCAAAGTAGGATGATTTTATTTTTAGGTGAGTAGAGGGAGGTTGAGCAGTCAAGTAGT
      CCCGTTTCATCCTACTAAAATAAAAATCCACTCATCTCCCTCCAACTCGTCAGTTCATCA

°901  ACAGAGAAATGTGGAATATAAACATAAGGAGAGTCAGTCAGTCACAAATATTTATGGAAC
      TGTCTCTTTACACCTTATATTTGTATTCCTCTCAGTCAGTCAGTGTTTATAAATACCTTG

9961  ACCTACTACATGCCAGACACTTTTGTACACATTGGAGTGCAGCACAGCAGGAGCTTATAT
      TGGATGATGTACGGTCTGTGAAAACATGTGTAACCTCACGTCGTGTCGTCCTCGAATATA

0021  TCTATTGGAAAGAGCAAGAGACAAGTTAGTACAAAACTAAATAAGGCAATCTCAATTGTG
      AGATAACCTTTCTCGTTCTCTGTTCAATCATGTTTTGATTTATTCCGTTAGAGTTAACAC

0081  TTGTTCAGAAGATAGAATAGAATAATATGGTAGAGAGAATAATATGATAGAGTAATACGT
      AACAAGTCTTCTATCTTATCTTATTATACCATCTCTCTTATTATACTATCTCATTATGCA

      10088 mbo11,

0141  AGAGTGTTACATTAGCTGTGGTGGTCATAGAAATCATCTTTGAGGAGTTGAGACCTAAAT
      TCTCACAATGTAATCGACACCACCAGTATCTTTAGTAGAAACTCCTCAACTCTGGATTTA

0201  GATGATAACAGGCCAGCTGATGGAGCTCTGAGAGAAGATGTTTCCAGACAGAGGGAAGAG
      CTACTATTGTCCGGTCGACTACCTCGAGACTCTCTTCTACAAAGGTCTGTCTCCCTTCTC

      10214 pvu11, 10223 sac1, 10234 mbo11, 10255 mbo11,

0261  AAAATGCAAAGGCCCTGAGAATGGAAAAAGTTTACCATGTTTGAGGAACAAGAAGGGACA
      TTTTACGTTTCCGGGACTCTTACCTTTTTCAAATGGTACAAACTCCTTGTTCTTCCCTGT

:0321  AGCATGACTTGTCACTTTCCCACCTTGCTTTCTAGCTCAAGTAAGCCCTTATTGCTGCAT
      TCGTACTGAACAGTGAAAGGGTGGAACGAAAGATCGAGTTCATTCGGGAATAACGACGTA
```

0381 TGCAACCATAGACTATTTCCCTAGTCCACTGACTTTCTCATTCTCCTGGACCTCTATCTT
ACGTTGGTATCTGATAAAGGGATCAGGTGACTGAAAGAGTAAGAGGACCTGGAGATAGAA

0441 AGATTTTCTGTGTTTTCCTTAAATCTCGAGCACCTTTTTTCCATTCTCCAATGCCCATTT
TCTAAAAGACACAAAAGGAATTTAGAGCTCGTGGAAAAAAGGTAAGAGGTTACGGGTAAA

10465 ava1 xho1,

0501 TCACTCTTAGCTGATGCTGTGCAATGAACCTCCACAGACTCCCAACATCACATTTACTGG
AGTGAGAATCGACTACGACACGTTACTTGGAGGTGTCTGAGGGTTGTAGTGTAAATGACC

0561 CATTTGTACCCATATACTCTGCCTGCACACTTATTACAATAAATAGAGTGTCTGTGCTCC
GTAAACATGGGTATATGAGACGGACGTGTGAATAATGTTATTTATCTCACAGACACGAGG

10619 mstII,

0621 TAAGGCCAACCTCTCCAGTTTTGTACTTGATTCCCATCTCTTCTCCCTTCATAAAAAATG
ATTCCGGTTGGAGAGGTCAAAACATGAACTAAGGGTAGAGAAGAGGGAAGTATTTTTTAC

10659 mbo11,

0681 TTGCTCCAGCAATTTCTCCTTTCTCCCTTACATCATCAATTTATCTCTCCACTAGATTAA
AACGAGGTCGTTAAAGAGGAAAGAGGGAATGTAGTAGTTAAATAGAGAGGTGATCTAATT

0741 TCTCATCAACATCAATCATGCTATTATTTCCTCCCCTATCTTTACAGAAACCGTCTTAAA
AGAGTAGTTGTAGTTAGTACGATAATAAAGGAGGGGATAGAAATGTCTTTGGCAGAATTT

0801 ATAATTGTCCCTGCTTGCAGCCTTTGCATCTTTTCCTGCCATTCTCTCCTCTTTAACCCA
TATTAACAGGGACGAACGTCGGAAACGTAGAAAAGGACGGTAAGAGAGGAGAAATTGGGT

0861 CTCCAAAGAAGCTTTTATCTCTATTATTTCATAAGAACTACATTTTCAAGGTCATTATTG
GAGGTTTCTTCGAAAATAGAGATAATAAAGTATTCTTGATGTAAAAGTTCCAGTAATAAC

10869 hind111,

0921 ATCTTTACATTACTGATTACTAGCCCTCATCTTATTTGAATTCTCAGTAGTATTTGAGAG EcoRI #76
TAGAAATGTAATGACTAATGATCGGGAGTAGAATAAACTTAAGAGTCATCATAAACTCTC

10958 ecor1,

0981 GTGATTACTCCATCGTTGATACAGGTTCTCACTTGGTTTCTCTAACACCACTTTTCTTCA
CACTAATGAGGTAGCAACTATGTCCAAGAGTGAACCAAAGAGATTGTGGTGAAAAGAAGT

11035 mbo11,

1041 CTTTTCTACTAAACTTACTGCCAATCTTTGTCAACTTTGCGAGTCCTTCTTCATCCCCCT
GAAAAGATGATTTGAATGACGGTTAGAAACAGTTGAAACGCTCAGGAAGAAGTAGGGGGA

11088 mbo11,

1101 AATCTTTAAAGGTTAGAGTGCTCCAGGGATTAGTTCTCCAACTTCTTCTTTATTCTATTT
TTAGAAATTTCCAATCTCACGAGGTCCCTAATCAAGAGGTTGAAGAAGAAATAAGATAAA

11105 aha111, 11144 mbo11,

1161 ATACTCACTCTGTGACGACATTGTTTAGCCTCATGATTTTAAGTACTATCTATCTGCAGA
TATGAGTGAGACACTGCTGTAACAAATCGGAGTACTAAAATTCATGATAGATAGACGTCT

11202 sca1, 11214 pst1,

1221 TAATTCCAGTTGAATATCTGGAGATATTCTCAGATCTCTCTCCTGAAGTCAAAATTTACA
ATTAAGGTCAACTTATAGACCTCTATAAGAGTCTAGAGAGAGGACTTCAGTTTTAAATGT

11252 bgl11,

1281 TATCAACTCCCTACCTTAACATGCACACAATTAGACTCTTAATTCCTGCCCCTTCCCAAA
ATAGTTGAGGGATGGAATTGTACGTGTGTTAATCTGAGAATTAAGGACGGGGAAGGGTTT

1341 ACCTGCTGATTGCTCAGCCTTCTCCATGGCAATCAAAAACCATTCCTGGCCGGGCATGGT
TGGACGACTAACGAGTCGGAAGAGGTACCGTTAGTTTTTGGTAAGGACCGGCCCGTACCA

11364 nco1,

1401 GGCTCACTCCTGTAACCCCAGCACTTGGGGAAGCTGAGGCAGGTGGATTGGTTGAGCCCC
CCGAGTGAGGACATTGGGGTCGTGAACCCCTTCGACTCCGTCCACCTAACCAACTCGGGG

11434 tthIII1,

1461 AGAGTTCAAGATCAGCCTGGGCAACATGGCAAAACCTCATCTCTATTAAAAATACAAAAA
TCTCAAGTTCTAGTCGGACCCGTTGTACCGTTTTGGAGTAGAGATAATTTTTATGTTTTT

```
1521  TTAGCCAGGCGTGGTGGTGCACACCTGCAGTCCCAGCTACTCAGGAGGCTGAGGCACGAG
      AATCGGTCCGCACCACCACGTGTGGACGTCAGGGTCGATGAGTCCTCCGACTCCGTGCTC

      11545 pst1,

1581  AATCGCTTGAACTGGGGAGACGGAGGTTGCAGTGAGCTGAGATGGCCTCACTGCACTCCA
      TTAGCGAACTTGACCCCTCTGCCTCCAACGTCACTCGACTCTACCGGAGTGACGTGAGGT

1641  GCCTGGGCAACAGAGTCAGACTTGATCTCAAAAAAATAAATAAGTAAAACCATTCCCTAT
      CGGACCCGTTGTCTCAGTCTGAACTAGAGTTTTTTTATTTATTCATTTTGGTAAGGGATA

1701  TTCAGTTATTCAGTCACAGATCTTGAAGTTTTCCAACTCTTTTTTTCTTACATTAACATC
      AAGTCAATAAGTCAGTGTCTAGAACTTCAAAAGGTTGAGAAAAAAAGAATGTAATTGTAG

      11718 bgl11,

1761  TAATCTGTAAAGGATCCTGTATTAGTCCATTTTCACACTGCTAATAAAGACATACCTGAG
      ATTAGACATTTCCTAGGACATAATCAGGTAAAAGTGTGACGATTATTTCTGTATGGACTC

      11772 bamh1 binI,

1821  ACTGGGCAATTTACAAAAGAAAGAAGTTTAATGGACTCACAGTTCCACATGGCTGGGGAG
      TGACCCGTTAAATGTTTTCTTTCTTCAAATTACCTGAGTGTCAAGGTGTACCGACCCCTC

      11865 bstXI, 11879 stu1,

1881  GCCTCACAATCATGGCAGAAGGCAGGGAGGAGCAAGTTATATCTCACATGATGGCACAGG
      CGGAGTGTTAGTACCGTCTTCCGTCCCTCCTCGTTCAATATAGAGTGTACTACCGTGTCC

1941  CAAAGAGAGAGAGCTTGTGCAGGGAAACTCCTCTTTTTAAAACCATCAGATCTTGTGAGA
      GTTTCTCTCTCTCGAACACGTCCCTTTGAGGAGAAAAATTTTGGTAGTCTAGAACACTCT

      11976 aha111, 11988 bgl11,

2001  CTTATTCACTATCATGAGAACAGCAAGGGAAGGACCTACTGCCATGATTCAGTTACCTCC
      GAATAAGTGATAGTACTCTTGTCGTTCCCTTCCTGGATGACGGTACTAAGTCAATGGAGG

2061  TATCAGGTCCCTCCCACAGAACATGGGAATTCAAGATGAGATTTGGGTAGGGACACAGCC  EcoRI #17
      ATAGTCCAGGGAGGGTGTCTTGTACCCTTAAGTTCTACTCTAAACCCATCCCTGTGTCGG

      12087 ecor1,

2121  AAACCATATCATTCCACCCCATCCCCTCCCAAATCTCGTGTCCTCACATTTCAAAACCAA
      TTTGGTATAGTAAGGTGGGGTAGGGGAGGGTTTAGAGCACAGGAGTGTAAAGTTTTGGTT

2181  TCATGCCTTCCCAACAGTCCCCCAAAGTCATAACACATTTCAGCATTAACTCAAAAGTCC
      AGTACGGAAGGGTTGTCAGGGGGTTTCAGTATTGTGTAAAGTCGTAATTGAGTTTTCAGG

2241  ACAGTCCAATGTCTCATCTAAGACAAGGCAAGTCCCTTCCACCTATGAGCATGTAAAGTC
      TGTCAGGTTACAGAGTAGATTCTGTTCCGTTCAGGGAAGGTGGATACTCGTACATTTCAG

2301  AAAAGCAAGTTAGTTACTTCCTAGATACAATGGGGTATAGGCATTGGGTAAATACAGCCA
      TTTTCGTTCAATCAATGAAGGATCTATGTTACCCCATATCCGTAACCCATTTATGTCGGT

2361  TTCCAAATGGGAGAAATTGGCCAAAACAAAGGGCCTACAGGCCCCATGCAAGTCTGAAAT
      AAGGTTTACCCTCTTTAACCGGTTTTGTTTCCCGGATGTCCGGGGTACGTTCAGACTTTA

      12378 bal1,

2421  TCTACGGGGCAGTCAAATCTTAAATCTCTAAAGTGATCTCCTTTGACTCCATGTCTTGCA
      AGATGCCCCGTCAGTTTAGAATTTAGAGATTTCACTAGAGGAAACTGAGGTACAGAACGT

2481  TCTGAGTCATGCTGATGCAAGAGGTGGGTGCCCATGGTCTTGGGCAGCTCCACCCCTGTG
      AGACTCAGTACGACTACGTTCTCCACCCACGGGTACCAGAACCCGTCGAGGTGGGGACAC

      12512 bstXI nco1, 12530 bstXI,

2541  GCTTTGCAGGGTACAGCCTCCCTTGTGTCTGCCTTCATGGGCTGGCATTTTCTGTAGCTT
      CGAAACGTCCCATGTCGGAGGGAACACAGACGGAAGTACCCGACCGTAAAAGACATCGAA

2601  TTCCAGATGCTGGTGCAAGTTTCTGATGGATCTACCATTCCGGGGTCTGGAGGACGGTGG
      AAGGTCTACGACCACGTTCAAAGACTACCTAGATGGTAAGGCCCCAGACCTCCTGCCACC

      12628 binI,

2661  CCCTCTTCTCACAGCTCCACTAGGTGGTACCCCAGTAGGGACTCTGTGTGGGTGCCCTGA
      GGGAGAAGAGTGTCGAGGTGATCCACCATGGGGTCATCCCTGAGACACACCCACGGGACT

      12664 msc11, 12685 kpn1,
```

2721 GCCCACATTTCCCTTCTGCACTGCCCTAGCAGAGTTTCTACATGAAGGCCCACCCCCGCA
CGGGTGTAAAGGGAAGACGTGACGGGATCGTCTCAAAGATGTACTTCCGGGTGGGGGCGT

2781 GCAAACTTCTGCCTGGGCATCCAGGCACTTCCATTAATTTCTAAACAACAAAGCATTCAA
CGTTTGAAGACGGACCCGTAGGTCCGTGAAGGTAATTAAAGATTTGTTGTTTCGTAAGTT

2841 GAGGCGACTTGGGTGCTATTAAGGGCATTCAGTTTCATAAGGGAAGCAGAGCATAAAAGT
CTCCGCTGAACCCACGATAATTCCCGTAAGTCAAAGTATTCCCTTCGTCTCGTATTTTCA

2901 TTGGAAAATTTGCAGCCTGATAATGTTAGAGAAAAGCAAATCCCATTTTCTGAGGAGAAA
AACCTTTTAAACGTCGGACTATTACAATCTCTTTTCGTTTAGGGTAAAAGACTCCTCTTT

2961 TTCAAGCAGGCTGCAGAAATTTGCATAAGTAATGAGGAGCCGAATGTTAATCCCAAGACA
AAGTTCGTCCGACGTCTTTAAACGTATTCATTACTCCTCGGCTTACAATTAGGGTTCTGT

12971 pst1, 13013 bstXI,

3021 ATGGGGAAAATGTCTCCAGGGCATGTCAGAGGTCTTCACACCAGCCCCTCCCATCACCGG
TACCCCTTTTACAGAGGTCCCGTACAGTCTCCAGAAGTGTGGTCGGGGAGGGTAGTGGCC

13053 mbo11,

3081 CCCAGAGGCCTAAGAGGAAAAAGTTGTTTCATGGGCTGGGCCCAGGATCCCCATGCTGTG
GGGTCTCCGGATTCTCCTTTTTCAACAAAGTACCCGACCCGGGTCCTAGGGGTACGACAC

13086 stu1, 13118 apa1, 13125 bamh1 binI,

3141 TGCAGCCTAGGGAGTCGGTGCCTTGTGTCCCAGCCGCTACAGCCATGGCTGAAAGGGGTC
ACGTCGGATCCCTCAGCCACGGAACACAGGGTCGGCGATGTCGGTACCGACTTTCCCCAG

13146 avr2, 13183 nco1,

3201 AATGTAGAGCTCAGGCTGTGGCTTCATAGGGTACAAGTCCCAAGCCTTGGCAGCTTCCAC
TTACATCTCGAGTCCGACACCGAAGTATCCCATGTTCAGGGTTCGGAACCGTCGAAGGTG

13207 sac1,

3261 ATGATGTTGAGCCTGCGAGTGCACAGAAGTCAAGAACTGGGGTTTGGGAACTTCCGCCTA
TACTACAACTCGGACGCTCACGTGTCTTCAGTTCTTGACCCCAAACCCTTGAAGGCGGAT

3321 GATTTCAGAAGATGTATGAAAACACCTGGATGCCTAGGCAGAAGTTTGCTGCGGGGGTGG
CTAAAGTCTTCTACATACTTTTGTGGACCTACGGATCCGTCTTCAAACGACGCCCCCACC

13328 mbo11, 13353 avr2, 13379 apa1,

3381 GCCCTCATGGAGAACCTCTGCTAGGGCAGTGCAGAAGGGAAATGTGGGCTCAGAGCACAC
CGGGAGTACCTCTTGGAGACGATCCCGTCACGTCTTCCCTTTACACCCGAGTCTCGTGTG

3441 ACACAGAGTCCCTGCTGGGGTACCACCTAGTGGAGCTGTGAGAAGAGGACCACCGTCCTC
TGTGTCTCAGGGACGACCCCATGGTGGATCACCTCGACACTCTTCTCCTGGTGGCAGGAG

13459 kpn1, 13482 mbo11,

3501 CAGGCCCCAGAATGGTAGATACCCTAAATCATCTCTTTCAAATTCGAAGTTCCACAAATC
GTCCGGGGTCTTACCATCTATGGGATTTAGTAGAGAAAGTTTAAGCTTCAAGGTGTTTAG

3561 TCTAGGACAGGAGCAAAATGCCACCAGTATCTTTGCTAAAACATAACAAGAGTTACCTTT
AGATCCTGTCCTCGTTTTACGGTGGTCATAGAAACGATTTTGTATTGTTCTCAATGGAAA

3621 GCTCCAGTTCCCAACAAGTTCCTCATCTCTATCTGAGACCACCTCAGCCTGGATTTCATT
CGAGGTCAAGGGTTGTTCAAGGAGTAGAGATAGACTCTGGTGGAGTCGGACCTAAAGTAA

3681 GTCCATATCATTATCAGCCTTTTGGTCAAAGCCATTCAACAAGTCTCTAGAGAGTTCCAA
CAGGTATAGTAATAGTCGGAAAACCAGTTTCGGTAAGTTGTTCAGAGATCTCTCAAGGTT

13726 xba1,

3741 ACTTTCCACATTTTCCTGTCGTCTTCTGAGCCCTCCAAACTGTTCCAACCTCTGCCTGTT
TGAAAGGTGTAAAAGGACAGCAGAAGACTCGGGAGGTTTGACAAGGTTGGAGACGGACAA

13762 mbo11,

3801 ACCCAGTTCCAAAGTCGCTTCCACATTTTTGGGTATCTTTTCAGCAGCACCCCACTCTAC
TGGGTCAAGGTTTCAGCGAAGGTGTAAAAACCCATAGAAAAGTCGTCGTGGGGTGAGATG

13821 bstXI, 13852 bstXI,

3861 TGGTACCAACTTACTGTATTAGTCTGTTCTCACACTGCTGATAAAGATATACCTGAGACT
ACCATGGTTGAATGACATAATCAGACAAGAGTGTGACGACTATTTCTATATGGACTCTGA

[illegible]

```
13921  GGGAAATTTACAAAGGAAAGAGGTTTTATGGACTTACTGTTCCACATGGCTGGGGAGGCC
       CCCTTTAAATGTTTCCTTTCTCCAAAATACCTGAATGACAAGGTGTACCGACCCCTCCGG
       13962 bstXI, 13976 stu1,
13981  TCAAAATCATGGCAGAAGGCAAGGAGGAGCAAGTCTTGTCTTACATGGATGGCAGCAGGC
       AGTTTTAGTACCGTCTTCCGTTCCTCCTCGTTCAGAACAGAATGTACCTACCGTCGTCCG
14041  AAAGAAAGAAAGCTTGTGCAGGAAAACTCTTTTTTTTCTTTTCTTCTTTTTAGACAGAGA
       TTTCTTTCTTTCGAACACGTCCTTTTGAGAAAAAAAAGAAAAGAAGAAAAATCTGTCTCT
       14050 hind111, 14082 mbo11, 14098 bgl11,
14101  TCTTGCTCTGTCACGCAGGCTGGAGTCAGTGGCGTGAGTCTTGGCTCACTGCAACCTCCA
       AGAACGAGACAGTGCGTCCGACCTCAGTCACCGCACTCAGAACCGAGTGACGTTGGAGGT
14161  CCTCCCGGGTTCAAGTGATTCTCCTGCCTCAGCCTTCCAAGTAGCTGGTACTACAGGCAT
       GGAGGGCCCAAGTTCACTAAGAGGACGGAGTCGGAAGGTTCATCGACCATGATGTCCGTA
       14164 ava1 sma1 xma1, 14184 tthIII1, 14197 bstXI,
14221  GTGCCACCACATGAGATGAGATTTTGGTAGGGACACAGCCAAACCATATCATTCCCCCTG
       CACGGTGGTGTACTCTACTCTAAAACCATCCCTGTGTCGGTTTGGTATAGTAAGGGGGAC
14281  TCCCCTCCCAAATCTCATGTCCTCACATTTCAAAACCAATCATGCCTTCCCAACAGTCCC
       AGGGGAGGGTTTAGAGTACAGGAGTGTAAAGTTTTGGTTAGTACGGAAGGGTTGTCAGGG
14341  CCAAAGTCATAACTCATTTCAGCATTAACTCAAAAGTCCACAGTATTTTTAGTAGAGATG
       GGTTTCAGTATTGAGTAAAGTCGTAATTGAGTTTTCAGGTGTCATAAAAATCATCTCTAC
14401  GGGTTTCACCATATTGGCCAGACTGGTCTCTAACTCCTGACCTTATGATCCGCCCGCCTT
       CCCAAAGTGGTATAACCGGTCTGACCAGAGATTGAGGACTGGAATACTAGGCGGGCGGAA
       14415 bal1,
14461  GGCCTCCCAAAGCACTGGGATTACAGGTGTGAGCCACCATGCCCAGCCAAAAACTCTTCT
       CCGGAGGGTTTCGTGACCCTAATGTCCACACTCGGTGGTACGGGTCGGTTTTTGAGAAGA
       14467 bstXI, 14515 mbo11,
14521  TTTTAAAACCATCAGTTCTCGATGAGACTTATTCACTATCACAAGAACAGCATAGGAAAG
       AAAATTTTGGTAGTCAAGAGCTACTCTGAATAAGTGATAGTGTTCTTGTCGTATCCTTTC
       14522 aha111, 14579 bgl11,
14581  ATCTGCCCCCATGATTCAATTACCTCCCACCAGGTCCCTCCCACAACACATGGGAATTCA   EcoRI #55
       TAGACGGGGGTACTAAGTTAATGGAGGGTGGTCCAGGGAGGGTGTTGTGTACCCTTAAGT
       14634 ecor1,
14641  CGATGAGATTTGGGTGGGGATCCTCTTGGCTATCATTTCAAAAAATAGTCAGGGTTGATT
       GCTACTCTAAACCCACCCCTAGGAGAACCGATAGTAAAGTTTTTTATCAGTCCCAACTAA
       14658 bamhi binl,
14701  ATGGACTTAGTATTGAATAAGATGAAAATGTACTGAAATGGAACGATGAAGATAATTGAC
       TACCTGAATCATAACTTATTCTACTTTTACATGACTTTACCTTGCTACTTCTATTAACTG
       14748 mbo11,
14761  CAGGGAAAGCAGGTTACAAGCCAAGAGAGGATACAGTGTCACTTCAGATGCTATAAAAGT
       GTCCCTTTCGTCCAATGTTCGGTTCTCTCCTATGTCACAGTGAAGTCTACGATATTTTCA
14821  GATGTTTTTATTTTTTGAATGTTATTTGGCTATATTTTCAAATTATCTGCACTGCATATG
       CTACAAAAATAAAAAACTTACAATAAACCGATATAAAAGTTTAATAGACGTGACGTATAC
       14875 nde1,
14881  CACTGCTTCTGTAATAATAAAAGGGTATTAAAATAATAAAATCAAGTCTATAAATCACAC
       GTGACGAAGACATTATTATTTTCCCATAATTTTATTATTTTAGTTCAGATATTTAGTGTG
14941  ACACACACCCCAGCCTCTTCTCTACACCTCAGTGACTACCAACCACATCTAAATTATCAT
       TGTGTGTGGGGTCGGAGAAGAGATGTGGAGTCACTGATGGTTGGTGTAGATTTAATAGTA
       14956 mbo11,
15001  CATTTCTCCATCTGAATTATTGCAAGAAGCCATTGCAATAATTCAGATGAGAGTTGATACA
       GTAAAGAGTAGACTTAATAACGTTCTTCGGTAACGTTATTAAGTCTACTCTCAACTATGT
```

```
:5061   AAGCCTTCCTGTTTCCAACCTTACCCTCATATAGTCTTTTCCCAACATTGCATCTAAAGT
        TTCGGAAGGACAAAGGTTGGAATGGGAGTATATCAGAAAAGGGTTGTAACGTAGATTTCA

.5121   AATTTTTCAAATTGCAATTCTGATCCGGGGAATTC
        TTAAAAAGTTTAACGTTAAGACTAGGCCCCTTAAG

        15150 ecor1,
```

APPENDIX B

```
     ValTyrGlyPheTrpGlyAlaThrThrGlnThrPheGlySerArgGlyMetThrAlaLeu
  1  GTCTATGGATTCTGGGGTGCCACAACTCAGACTTTCGGAAGCAGAGGCATGACCGCCTTA
     CAGATACCTAAGACCCCACGGTGTTGAGTCTGAAAGCCTTCGTCTCCGTACTGGCGGAAT

     LeuLysValSerSerCysAspLysAsnThrGlyAspTyrTyrGluAspSerTyrGluAsp
 61  CTGAAGGTTTCTAGTTGTGACAAGAACACTGGTGATTATTACGAGGACAGTTATGAAGAT
     GACTTCCAAAGATCAACACTGTTCTTGTGACCACTAATAATGCTCCTGTCAATACTTCTA

     115 mbo11,

     IleSerAlaTyrLeuLeuSerLysAsnAsnAlaIleGluProArgSerPheSerGlnAsn
121  ATTTCAGCATACTTGCTGAGTAAAAACAATGCCATTGAACCAAGAAGCTTCTCCCAGAAT
     TAAAGTCGTATGAACGACTCATTTTTGTTACGGTAACTTGGTTCTTCGAAGAGGGTCTTA

     165 hind111, 177 ecor1,

     SerArgHisProSerThrArgGlnLysGlnPheAsnAlaThrThrIleProGluAsnAsp
181  TCAAGACACCCTAGCACTAGGCAAAAGCAATTTAATGCCACCACAATTCCAGAAAATGAC
     AGTTCTGTGGGATCGTGATCCGTTTTCGTTAAATTACGGTGGTGTTAAGGTCTTTTACTG

     IleGluLysThrAspProTrpPheAlaHisArgThrProMetProLysIleGlnAsnVal
241  ATAGAGAAGACTGACCCTTGGTTTGCACACAGAACACCTATGCCTAAAATACAAAATGTC
     TATCTCTTCTGACTGGGAACCAAACGTGTGTCTTGTGGATACGGATTTTATGTTTTACAG

     246 mbo11,

     SerSerSerAspLeuLeuMetLeuLeuArgGlnSerProThrProHisGlyLeuSerLeu
301  TCCTCTAGTGATTTGTTGATGCTCTTGCGACAGAGTCCTACTCCACATGGGCTATCCTTA
     AGGAGATCACTAAACAACTACGAGAACGCTGTCTCAGGATGAGGTGTACCCGATAGGAAT

     329 tthIII1,

     SerAspLeuGlnGluAlaLysTyrGluThrPheSerAspAspProSerProGlyAlaIle
361  TCTGATCTCCAAGAAGCCAAATATGAGACTTTTTCTGATGATCCATCACCTGGAGCAATA
     AGACTAGAGGTTCTTCGGTTTATACTCTGAAAAAGACTACTAGGTAGTGGACCTCGTTAT

     AspSerAsnAsnSerLeuSerGluMetThrHisPheArgProGlnLeuHisHisSerGly
421  GACAGTAATAACAGCCTGTCTGAAATGACACACTTCAGGCCACAGCTCCATCACAGTGGG
     CTGTCATTATTGTCGGACAGACTTTACTGTGTGAAGTCCGGTGTCGAGGTAGTGTCACCC

     468 bstXI,

     AspMetValPheThrProGluSerGlyLeuGlnLeuArgLeuAsnGluLysLeuGlyThr
481  GACATGGTATTTACCCCTGAGTCAGGCCTCCAATTAAGATTAAATGAGAAACTGGGGACA
     CTGTACCATAAATGGGGACTCAGTCCGGAGGTTAATTCTAATTTACTCTTTGACCCCTGT

     497 tthIII1, 504 stu1,

     ThrAlaAlaThrGluLeuLysLysLeuAspPheLysValSerSerThrSerAsnAsnLeu
541  ACTGCAGCAACAGAGTTGAAGAAACTTGATTTCAAAGTTTCTAGTACATCAAATAATCTG
     TGACGTCGTTGTCTCAACTTCTTTGAACTAAAGTTTCAAAGATCATGTAGTTTATTAGAC

     542 pst1, 558 mbo11,

     IleSerThrIleProSerAspAsnLeuAlaAlaGlyThrAspAsnThrSerSerLeuGly
601  ATTTCAACAATTCCATCAGACAATTTGGCAGCAGGTACTGATAATACAAGTTCCTTAGGA
     TAAAGTTGTTAAGGTAGTCTGTTAAACCGTCGTCCATGACTATTATGTTCAAGGAATCCT

     653 mstII,

     ProProSerMetProValHisTyrAspSerGlnLeuAspThrThrLeuPheGlyLysLys
661  CCCCCAAGTATGCCAGTTCATTATGATAGTCAATTAGATACCACTCTATTTGGCAAAAAG
     GGGGGTTCATACGGTCAAGTAATACTATCAGTTAATCTATGGTGAGATAAACCGTTTTTC

     SerSerProLeuThrGluSerGlyGlyProLeuSerLeuSerGluGluAsnAsnAspSer
721  TCATCTCCCCTTACTGAGTCTGGTGGACCTCTGAGCTTGAGTGAAGAAAATAATGATTCA
     AGTAGAGGGGAATGACTCAGACCACCTGGAGACTCGAACTCACTTCTTTTATTACTAAGT

     763 mbo11,

     LysLeuLeuGluSerGlyLeuMetAsnSerGlnGluSerSerTrpGlyLysAsnValSer
781  AAGTTGTTAGAATCAGGTTTAATGAATAGCCAAGAAAGTTCATGGGGAAAAAATGTATCG
     TTCAACAATCTTAGTCCAAATTACTTATCGGTTCTTTCAAGTACCCCTTTTTTACATAGC

     SerThrGluSerGlyArgLeuPheLysGlyLysArgAlaHisGlyProAlaLeuLeuThr
841  TCAACAGAGAGTGGTAGGTTATTTAAAGGGAAAAGAGCTCATGGACCTGCTTTGTTGACT
     AGTTGTCTCTCACCATCCAATAAATTTCCCTTTTCTCGAGTACCTGGACGAAACAACTGA

     862 aha111, 875 sac1,
```

```
     LysAspAsnAlaLeuPheLysValSerIleSerLeuLeuLysThrAsnLysThrSerAsn
901  AAAGATAATGCCTTATTCAAAGTTAGCATCTCTTTGTTAAAGACAAACAAAACTTCCAAT
     TTTCTATTACGGAATAAGTTTCAATCGTAGAGAAACAATTTCTGTTTGTTTTGAAGGTTA

     AsnSerAlaThrAsnArgLysThrHisIleAspGlyProSerLeuLeuIleGluAsnSer
961  AATTCAGCAACTAATAGAAAGACTCACATTGATGGCCCATCATTATTAATTGAGAATAGT
     TTAAGTCGTTGATTATCTTTCTGAGTGTAACTACCGGGTAGTAATAATTAACTCTTATCA

     ProSerValTrpGlnAsnIleLeuGluSerAspThrGluPheLysLysValThrProLeu
1021 CCATCAGTCTGGCAAAATATATTAGAAAGTGACACTGAGTTTAAAAAAGTGACACCTTTG
     GGTAGTCAGACCGTTTTATATAATCTTTCACTGTGACTCAAATTTTTTCACTGTGGAAAC

     1021 bstXI, 1060 aha111,

     IleHisAspArgMetLeuMetAspLysAsnAlaThrAlaLeuArgLeuAsnHisMetSer
1081 ATTCATGACAGAATGCTTATGGACAAAAATGCTACAGCTTTGAGGCTAAATCATATGTCA
     TAAGTACTGTCTTACGAATACCTGTTTTTACGATGTCGAAACTCCGATTTAGTATACAGT

     1132 nde1,

     AsnLysThrThrSerSerLysAsnMetGluMetValGlnGlnLysLysGluGlyProIle
1141 AATAAAACTACTTCATCAAAAAACATGGAAATGGTCCAACAGAAAAAAGAGGGCCCCATT
     TTATTTTGATGAAGTAGTTTTTTGTACCTTTACCAGGTTGTCTTTTTTCTCCCGGGGTAA

     1191 apa1,

     ProProAspAlaGlnAsnProAspMetSerPhePheLysMetLeuPheLeuProGluSer
1201 CCACCAGATGCACAAAATCCAGATATGTCGTTCTTTAAGATGCTATTCTTGCCAGAATCA
     GGTGGTCTACGTGTTTTAGGTCTATACAGCAAGAAATTCTACGATAAGAACGGTCTTAGT

     AlaArgTrpIleGlnArgThrHisGlyLysAsnSerLeuAsnSerGlyGlnGlyProSer
1261 GCAAGGTGGATACAAAGGACTCATGGAAAGAACTCTCTGAACTCTGGGCAAGGCCCCAGT
     CGTTCCACCTATGTTTCCTGAGTACCTTTCTTGAGAGACTTGAGACCCGTTCCGGGGTCA

     ProLysGlnLeuValSerLeuGlyProGluLysSerValGluGlyGlnAsnPheLeuSer     cDNA clone
1321 CCAAAGCAATTAGTATCCTTAGGACCAGAAAAATCTGTGGAAGGTCAGAATTTCTTGTCT       C1
     GGTTTCGTTAATCATAGGAATCCTGGTCTTTTTAGACACCTTCCAGTCTTAAAGAACAGA

     1337 mstII,

     GluLysAsnLysValValValGlyLysGlyGluPheThrLysAspValGlyLeuLysGlu
1381 GAGAAAAACAAAGTGGTAGTAGGAAAGGGTGAATTTACAAAGGACGTAGGACTCAAAGAG
     CTCTTTTTGTTTCACCATCATCCTTTCCCACTTAAATGTTTCCTGCATCCTGAGTTTCTC

     MetValPheProSerSerArgAsnLeuPheLeuThrAsnLeuAspAsnLeuHisGluAsn
1441 ATGGTTTTTCCAAGCAGCAGAAACCTATTTCTTACTAACTTGGATAATTTACATGAAAAT
     TACCAAAAAGGTTCGTCGTCTTTGGATAAAGAATGATTGAACCTATTAAATGTACTTTTA

     AsnThrHisAsnGlnGluLysLysIleGlnGluGluIleGluLysLysGluThrLeuIle
1501 AATACACACAATCAAGAAAAAAAAATTCAGGAAGAAATAGAAAAGAAGGAAACATTAATC
     TTATGTGTGTTAGTTCTTTTTTTTTAAGTCCTTCTTTATCTTTTCTTCCTTTGTAATTAG

     1531 mbo11,

     GlnGluAsnValValLeuProGlnIleHisThrValThrGlyThrLysAsnPheMetLys
1561 CAAGAGAATGTAGTTTTGCCTCAGATACATACAGTGACTGGCACTAAGAATTTCATGAAG
     GTTCTCTTACATCAAAACGGAGTCTATGTATGTCACTGACCGTGATTCTTAAAGTACTTC

     1617 mbo11, 1620 xmn1,

     AsnLeuPheLeuLeuSerThrArgGlnAsnValGluGlySerTyrAspGlyAlaTyrAla
1621 AACCTTTTCTTACTGAGCACTAGGCAAAATGTAGAAGGTTCATATGACGGGGCATATGCT
     TTGGAAAAGAATGACTCGTGATCCGTTTTACATCTTCCAAGTATACTGCCCCGTATACGA

     1661 nde1, 1673 nde1,

     ProValLeuGlnAspPheArgSerLeuAsnAspSerThrAsnArgThrLysLysHisThr
1681 CCAGTACTTCAAGATTTTAGGTCATTAAATGATTCAACAAATAGAACAAAGAAACACACA
     GGTCATGAAGTTCTAAAATCCAGTAATTTACTAAGTTGTTTATCTTGTTTCTTTGTGTGT

     1683 sca1,

     AlaHisPheSerLysLysGlyGluGluGluAsnLeuGluGlyLeuGlyAsnGlnThrLys
1741 GCTCATTTCTCAAAAAAAGGGGAGGAAGAAAACTTGGAAGGCTTGGGAAATCAAACCAAG
     CGAGTAAAGAGTTTTTTTCCCCTCCTTCTTTTGAACCTTCCGAACCCTTTAGTTTGGTTC

     1765 mbo11,
```

```
      GlnIleValGluLysTyrAlaCysThrThrArgIleSerProAsnThrSerGlnGlnAsn
1801  CAAATTGTAGAGAAATATGCATGCACCACAAGGATATCTCCTAATACAAGCCAGCAGAAT
      GTTTAACATCTCTTTATACGTACGTGGTGTTCCTATAGAGGATTATGTTCGGTCGTCTTA

      1817 ava3, 1819 sph1, 1833 ecor5,

      PheValThrGlnArgSerLysArgAlaLeuLysGlnPheArgLeuProLeuGluGluThr
1861  TTTGTCACGCAACGTAGTAAGAGAGCTTTGAAACAATTCAGACTCCCACTAGAAGAAACA
      AAACAGTGCGTTGCATCATTCTCTCGAAACTTTGTTAAGTCTGAGGGTGATCTTCTTTGT

      1890 xmn1, 1912 mbo11,

      GluLeuGluLysArgIleIleValAspAspThrSerThrGlnTrpSerLysAsnMetLys
1921  GAACTTGAAAAAAGGATAATTGTGGATGACACCTCAACCCAGTGGTCCAAAAACATGAAA
      CTTGAACTTTTTTCCTATTAACACCTACTGTGGAGTTGGGTCACCAGGTTTTTGTACTTT

      HisLeuThrProSerThrLeuThrGlnIleAspTyrAsnGluLysGluLysGlyAlaIle
1981  CATTTGACCCCGAGCACCCTCACACAGATAGACTACAATGAGAAGGAGAAAGGGGCCATT
      GTAAACTGGGGCTCGTGGGAGTGTGTCTATCTGATGTTACTCTTCCTCTTTCCCCGGTAA

      1989 ava1,

      ThrGlnSerProLeuSerAspCysLeuThrArgSerHisSerIleProGlnAlaAsnArg
2041  ACTCAGTCTCCCTTATCAGATTGCCTTACGAGGAGTCATAGCATCCCTCAAGCAAATAGA
      TGAGTCAGAGGGAATAGTCTAACGGAATGCTCCTCAGTATCGTAGGGAGTTCGTTTATCT

      2098 bgl11,

      SerProLeuProIleAlaLysValSerSerPheProSerIleArgProIleTyrLeuThr
2101  TCTCCATTACCCATTGCAAAGGTATCATCATTTCCATCTATTAGACCTATATATCTGACC
      AGAGGTAATGGGTAACGTTTCCATAGTAGTAAAGGTAGATAATCTGGATATATAGACTGG

      ArgValLeuPheGlnAspAsnSerSerHisLeuProAlaAlaSerTyrArgLysLysAsp
2161  AGGGTCCTATTCCAAGACAACTCTTCTCATCTTCCAGCAGCATCTTATAGAAAGAAAGAT
      TCCCAGGATAAGGTTCTGTTGAGAAGAGTAGAAGGTCGTCGTAGAATATCTTTCTTTCTA

      2182 mbo11, 2190 mbo11,

      SerGlyValGlnGluSerSerHisPheLeuGlnGlyAlaLysLysAsnAsnLeuSerLeu
2221  TCTGGGGTCCAAGAAAGCAGTCATTTCTTACAAGGAGCCAAAAAAAATAACCTTTCTTTA
      AGACCCCAGGTTCTTTCGTCAGTAAAGAATGTTCCTCGGTTTTTTTTATTGGAAAGAAAT

      AlaIleLeuThrLeuGluMetThrGlyAspGlnArgGluValGlySerLeuGlyThrSer
2281  GCCATTCTAACCTTGGAGATGACTGGTGATCAAAGAGAGGTTGGCTCCCTGGGGACAAGT
      CGGTAAGATTGGAACCTCTACTGACCACTAGTTTCTCTCCAACCGAGGGACCCCTGTTCA

      2307 bcl1,

      AlaThrAsnSerValThrTyrLysLysValGluAsnThrValLeuProLysProAspLeu
2341  GCCACAAATTCAGTCACATACAAGAAAGTTGAGAACACTGTTCTCCCGAAACCAGACTTG
      CGGTGTTTAAGTCAGTGTATGTTCTTTCAACTCTTGTGACAAGAGGGCTTTGGTCTGAAC

      ProLysThrSerGlyLysValGluLeuLeuProLysValHisIleTyrGlnLysAspLeu
2401  CCCAAAACATCTGGCAAAGTTGAATTGCTTCCAAAAGTTCACATTTATCAGAAGGACCTA
      GGGTTTTGTAGACCGTTTCAACTTAACGAAGGTTTTCAAGTGTAAATAGTCTTCCTGGAT

      2422 xmn1,

      PheProThrGluThrSerAsnGlySerProGlyHisLeuAspLeuValGluGlySerLeu
2461  TTCCCTACGGAAACTAGCAATGGGTCTCCTGGCCATCTGGATCTCGTGGAAGGGAGCCTT
      AAGGGATGCCTTTGATCGTTACCCAGAGGACCGGTAGACCTAGAGCACCTTCCCTCGGAA

      2490 bal1, 2499 binI, 2520 mbo11,

      LeuGlnGlyThrGluGlyAlaIleLysTrpAsnGluAlaAsnArgProGlyLysValPro
2521  CTTCAGGGAACAGAGGGAGCGATTAAGTGGAATGAAGCAAACAGACCTGGAAAAGTTCCC
      GAAGTCCCTTGTCTCCCTCGCTAATTCACCTTACTTCGTTTGTCTGGACCTTTTCAAGGG

      PheLeuArgValAlaThrGluSerSerAlaLysThrProSerLysLeuLeuAspProLeu
2581  TTTCTGAGAGTAGCAACAGAAAGCTCTGCAAAGACTCCCTCCAAGCTATTGGATCCTCTT
      AAAGACTCTCATCGTTGTCTTTCGAGACGTTTCTGAGGGAGGTTCGATAACCTAGGAGAA

      2621 bstXI, 2631 bamh1 binI,

      AlaTrpAspAsnHisTyrGlyThrGlnIleProLysGluGluTrpLysSerGlnGluLys
2641  GCTTGGGATAACCACTATGGTACTCAGATACCAAAAGAAGAGTGGAAATCCCAAGAGAAG
      CGAACCCTATTGGTGATACCATGAGTCTATGGTTTTCTTCTCACCTTTAGGGTTCTCTTC

      2677 mbo11,
                                                                   cDNA clone
                                                                      2-11
```

```
      SerProGluLysThrAlaPheLysLysLysAspThrIleLeuSerLeuAsnAlaCysGlu
2701  TCACCAGAAAAAACAGCTTTTAAGAAAAAGGATACCATTTTGTCCCTGAACGCTTGTGAA
      AGTGGTCTTTTTTGTCGAAAATTCTTTTTCCTATGGTAAAACAGGGACTTGCGAACACTT

      SerAsnHisAlaIleAlaAlaIleAsnGluGlyGlnAsnLysProGluIleGluValThr
2761  AGCAATCATGCAATAGCAGCAATAAATGAGGGACAAAATAAGCCCGAAATAGAAGTCACC
      TCGTTAGTACGTTATCGTCGTTATTTACTCCCTGTTTTATTCGGGCTTTATCTTCAGTGG

      TrpAlaLysGlnGlyArgThrGluArgLeuCysSerGlnAsnProProValLeuLysArg
2821  TGGGCAAAGCAAGGTAGGACTGAAAGGCTGTGCTCTCAAAACCCACCAGTCTTGAAACGC
      ACCCGTTTCGTTCCATCCTGACTTTCCGACACGAGAGTTTTGGGTGGTCAGAACTTTGCG
               N-termini 80/77Kd

      HisGlnArgGluIleThrArgThrThrLeuGlnSerAspGlnGluGluIleAspTyrAsp
2881  CATCAACGGGAAATAACTCGTACTACTCTTCAGTCAGATCAAGAGGAAATTGACTATGAT
      GTAGTTGCCCTTTATTGAGCATGATGAGAAGTCAGTCTAGTTCTCCTTTAACTGATACTA

      2907 mbo11,

      AspThrIleSerValGluMetLysLysGluAspPheAspIleTyrAspGluAspGluAsn
2941  GATACCATATCAGTTGAAATGAAGAAGGAAGATTTTGACATTTATGATGAGGATGAAAAT
      CTATGGTATAGTCAACTTTACTTCTTCCTTCTAAAACTGTAAATACTACTCCTACTTTTA

      2961 mbo11, 2968 mbo11
              N-termini 70/67Kd

      GlnSerProArgSerPheGlnLysLysThrArgHisTyrPheIleAlaAlaValGluArg
3001  CAGAGCCCCCGCAGCTTTCAAAAGAAAACACGACACTATTTTATTGCTGCAGTGGAGAGG
      GTCTCGGGGGCGTCGAAAGTTTTCTTTTGTGCTGTGATAAAATAACGACGTCACCTCTCC

      3047 pst1,

      LeuTrpAspTyrGlyMetSerSerSerProHisValLeuArgAsnArgAlaGlnSerGly
3061  CTCTGGGATTATGGGATGAGTAGCTCCCCACATGTTCTAAGAAACAGGGCTCAGAGTGGC
      GAGACCCTAATACCCTACTCATCGAGGGGTGTACAAGATTCTTTGTCCCGAGTCTCACCG

      SerValProGlnPheLysLysValValPheGlnGluPheThrAspGlySerPheThrGln
3121  AGTGTCCCTCAGTTCAAGAAAGTTGTTTTCCAGGAATTTACTGATGGCTCCTTTACTCAG
      TCACAGGGAGTCAAGTTCTTTCAACAAAAGGTCCTTAAATGACTACCGAGGAAATGAGTC

      ProLeuTyrArgGlyGluLeuAsnGluHisLeuGlyLeuLeuGlyProTyrIleArgAla
3181  CCCTTATACCGTGGAGAACTAAATGAACATTTGGGACTCCTGGGGCCATATATAAGAGCA
      GGGAATATGGCACCTCTTGATTTACTTGTAAACCCTGAGGACCCCGGTATATATTCTCGT

      GluValGluAspAsnIleMetValThrPheArgAsnGlnAlaSerArgProTyrSerPhe
3241  GAAGTTGAAGATAATATCATGGTAACTTTCAGAAATCAGGCCTCTCGTCCCTATTCCTTC
      CTTCAACTTCTATTATAGTACCATTGAAAGTCTTTAGTCCGGAGAGCAGGGATAAGGAAG

      3247 mbo11, 3278 stu1,

      TyrSerSerLeuIleSerTyrGluGluAspGlnArgGlnGlyAlaGluProArgLysAsn
3301  TATTCTAGCCTTATTTCTTATGAGGAAGATCAGAGGCAAGGAGCAGAACCTAGAAAAAAC
      ATAAGATCGGAATAAAGAATACTCCTTCTAGTCTCCGTTCCTCGTCTTGGATCTTTTTTG

      3325 mbo11,

      PheValLysProAsnGluThrLysThrTyrPheTrpLysValGlnHisHisMetAlaPro    cDNA clone
3361  TTTGTCAAGCCTAATGAAACCAAAACTTACTTTTGGAAAGTGCAACATCATATGGCACCC       C2
      AAACAGTTCGGATTACTTTGGTTTTGAATGAAAACCTTTCACGTTGTAGTATACCGTGGG

      3409 nde1,

      ThrLysAspGluPheAspCysLysAlaTrpAlaTyrPheSerAspValAspLeuGluLys
3421  ACTAAAGATGAGTTTGACTGCAAAGCCTGGGCTTATTTCTCTGATGTTGACCTGGAAAAA
      TGATTTCTACTCAAACTGACGTTTCGGACCCGAATAAAGAGACTACAACTGGACCTTTTT

      AspValHisSerGlyLeuIleGlyProLeuLeuValCysHisThrAsnThrLeuAsnPro
3481  GATGTGCACTCAGGCCTGATTGGACCCCTTCTGGTCTGCCACACTAACACACTGAACCCT
      CTACACGTGAGTCCGGACTAACCTGGGGAAGACCAGACGGTGTGATTGTGTGACTTGGGA

      3492 stu1,

      AlaHisGlyArgGlnValThrValGlnGluPheAlaLeuPhePheThrIlePheAspGlu
3541  GCTCATGGGAGACAAGTGACAGTACAGGAATTTGCTCTGTTTTTCACCATCTTTGATGAG
      CGAGTACCCTCTGTTCACTGTCATGTCCTTAAACGAGACAAAAAGTGGTAGAAACTACTC

      ThrLysSerTrpTyrPheThrGluAsnMetGluArgAsnCysArgAlaProCysAsnIle
3601  ACCAAAAGCTGGTACTTCACTGAAAATATGGAAAGAAACTGCAGGGCTCCCTGCAATATC
      TGGTTTTCGACCATGAAGTGACTTTTATACCTTTCTTTGACGTCCCGAGGCACGTTATAG

      3639 pst1,
```

```
      GlnMetGluAspProThrPheLysGluAsnTyrArgPheHisAlaIleAsnGlyTyrIle
3661  CAGATGGAAGATCCCACTTTTAAAGAGAATTATCGCTTCCATGCAATCAATGGCTACATA
      GTCTACCTTCTAGGGTGAAAATTTCTCTTAATAGCGAAGGTACGTTAGTTACCGATGTAT

      3667 mboii, 3679 ahaiii,

      MetAspThrLeuProGlyLeuValMetAlaGlnAspGlnArgIleArgTrpTyrLeuLeu
3721  ATGGATACACTACCTGGCTTAGTAATGGCTCAGGATCAAAGGATTCGATGGTATCTGCTC
      TACCTATGTGATGGACCGAATCATTACCGAGTCCTAGTTTCCTAAGCTACCATAGACGAG

      3753 binI,

      SerMetGlySerAsnGluAsnIleHisSerIleHisPheSerGlyHisValPheThrVal
3781  AGCATGGGCAGCAATGAAAACATCCATTCTATTCATTTCAGTGGACATGTGTTCACTGTA
      TCGTACCCGTCGTTACTTTTGTAGGTAAGATAAGTAAAGTCACCTGTACACAAGTGACAT

      ArgLysLysGlu
3841  CGAAAAAAAGAG
      GCTTTTTTTCTC
```

## Claims

**1.** A recombinant nucleic acid molecule coding for full length human Factor VIII:C protein or a portion thereof that possesses anticoagulant activity, said nucleic acid molecule comprising:

a) the nucleotide sequence:

5' TTT CAA AAG AAA ACA CGA CAC TAT TTT ATT GCT GCA GTG GAG AGG CTC TGG GAT TAT GGG ATG 3';

or

b) a nucleotide sequence that is complementary to the sequence of part a).

**2.** A nucleic acid molecule according to claim 1, which encodes the amino acid sequence:

PHE-GLN-LYS-LYS-THR-ARG-HIS-TYR-PHE-ILE-ALA-ALA-VAL-GLU-ARG-LEU-TRP-ASP-TYR-GLY-MET.

**3.** A nucleic acid molecule according to claim 1 or claim 2, wherein the nucleic acid is DNA.

**4.** A nucleic acid molecule according to any one of the preceding claims comprising the expressed nucleotide sequence of the 14.43 kb *Eco*RI fragment of the human genomic Factor VIII:C insert of bacteriophage λFVIII23D (ATCC Accession No. 40094).

**5.** A nucleic acid molecule according to any one of claims 1-3 comprising the expressed nucleotide sequence of the *Eco*RI fragment of about 4.4 kb size of the human genomic Factor VIII:C insert of bacteriophage λFVIII23D (ATCC Accession No. 40094).

**6.** A vector comprising a nucleic acid molecule according to any preceding claim.

**7.** A vector according to claim 6, which is an expression vector.

**8.** A vector according to claim 6, which is a recombinant mammalian virus.

**9.** A method for generating a polypeptide comprising the amino acid sequence PHE-GLN-LYS-LYS-THR-ARG-HIS-TYR-PHE-ILE-ALA-ALA-VAL-GLU-ARG-LEU-TRP-ASP-TYR-GLY-MET, comprising introducing into a host cell a recombinant nucleic acid molecule according to any one of claims 1-5, or a vector according to any one of claims 6-8.

**10.** A host cell composition prepared by introducing into a host cell a nucleic acid molecule according to any one of claims 1-5, or a vector according to any one of claims 6-8 and growing said host cell.

**11.** A host cell composition according to claim 10, wherein said host cell is an *E. coli* host cell or a mammalian host cell.

**12.** A method of preparing a recombinant DNA vector, comprising introducing a nucleic acid molecule according to any one of claims 1 to 5 into a vector.

**13.** A method according to claim 12, further comprising introducing the recombinant nucleic acid vector into a host cell.

**14.** The method of claim 13, further comprising growing said host cell to provide a composition of host cells comprising said recombinant vector.

**15.** Use of a nucleic acid molecule according to any one of claim 1 to 5, or a vector according to any one of claims 6-8, in the manufacture of a medicament for the treatment or diagnosis of haemophilia A.

**16.** Use of a nucleic acid molecule according to any one of claim 1 to 5, or a vector according to any one of claims 6-8, in the expression of full length human Factor VIII:C protein or a portion thereof.

**Patentansprüche**

**1.** Rekombinantes Nucleinsäuremolekül, das ein menschliches Faktor VIII:C-Protein mit voller Länge oder einen Teil davon codiert, das eine anticoagulierende Aktivität besitzt, wobei das Nucleinsäuremolekül umfasst:

a) die Nucleotidsequenz:

5' TTT CAA AAG AAA ACA CGA CAC TAT TTT ATT GCT GCA GTG GAG AGG CTC TGG GAT TAT GGG ATG 3';

oder
b) eine Nucleotidsequenz, die komplementär zu der Sequenz in Teil a) ist.

**2.** Nucleinsäuremolekül nach Anspruch 1, das die folgende Aminosäuresequenz codiert:

PHE-GLN-LYS-LYS-THR-ARG-HIS-TYR-PHE-ILE-ALA-ALA-VAL-GLU-ARG-LEU-TRP-ASP-TYR-GLY-MET.

**3.** Nucleinsäuremolekül nach Anspruch 1 oder 2, wobei die Nucleinsäure DNA ist.

**4.** Nucleinsäuremolekül nach einem der vorhergehenden Ansprüche, das die exprimierte Nucleotidsequenz des 14,43 kb-*Eco*RI-Fragments des menschlichen Genomfaktor-VIII:C-Inserts des Bakteriophagen λFVIII23D (ATCC-Hinterlegungsnummer 40094) umfasst.

**5.** Nucleinsäuremolekül nach einem der Ansprüche 1 bis 3, das die exprimierte Nucleinsequenz des *Eco*RI-Fragments, mit einer Größe von etwa 4,4 kb, des menschlichen Genomfaktor-VIII:C-Inserts des Bakteriopagen λFVIII23D (ATCC Hinterlegungsnummer 40094) umfasst.

**6.** Vektor, der ein Nucleinsäuremolekül nach einem der vorhergehenden Ansprüche umfasst.

**7.** Vektor nach Anspruch 6, der ein Expressionsvektor ist.

**8.** Vektor nach Anspruch 6, der ein rekombinantes Säugervirus ist.

**9.** Verfahren zur Herstellung eines Polypeptids, das die Aminosäuresequenz PHE-GLN-LYS-LYS-THR-ARG-HIS-TYR-PHE-ILE-ALA-ALA-VAL-GLU-ARG-LEU-TRP-ASP-TYR-GLY-MET umfasst, umfassend das Einführen eines rekombinanten Nucleinsäuremoeküls nach einem der Ansprüche 1 bis 5 oder eines Vektors nach einem der Ansprüche 6 bis 8 in eine Wirtszelle.

**10.** Wirtszellzusammensetzung, die hergestellt wird durch das Einführen eines Nucleinsäuremoleküls nach einem der Ansprüche 1 bis 5 oder eines Vektors nach einem der Ansprüche 6 bis 8 in eine Wirtszelle und das Züchten der Wirtszelle.

**11.** Wirtszellzusammensetzung nach Anspruch 10, wobei die Wirtszelle eine *E. coli*-Wirtszelle oder eine Säugerwirtszelle ist.

**12.** Verfahren zur Herstellung eines rekombinanten DNA-Vektors, das das Einführen eines Nucleinsäuremoleküls nach einem der Ansprüche 1 bis 5 in einen Vektor umfasst.

**13.** Verfahren nach Anspruch 12, das außerdem das Einführen des rekombinanten Nucleinsäurevektors in eine Wirtszelle umfasst.

**14.** Verfahren nach Anspruch 13, das außerdem das Züchten der Wirtszelle umfasst, um eine Zusammensetzung von Wirtszellen zu erhalten, die den rekombinanten Vektor umfassen.

**15.** Verwendung eines Nukleinsäuremoleküls nach einem der Ansprüche 1 bis 5 oder eines Vektors nach einem der Ansprüche 6 bis 8 für die Herstellung eines Arzneimittels für die Behandlung oder die Diagnose von Hämophilie A.

**16.** Verwendung eines Nucleinsäuremoleküls nach einem der Ansprüche 1 bis 5 oder eines Vektors nach einem der Ansprüche 6 bis 8 für die Exprimierung eines menschlichen Faktor VIII:C-Proteins mit voller Länge oder eines Teils davon.

**Revendications**

**1.** Molécule d'acide nucléique recombinante codant pour une protéine Facteur VIII:C humaine de pleine longueur, ou une partie de celle-ci, qui possède une activité anticoagulante, ladite molécule d'acide nucléique comportant :

a) la séquence nucléotidique suivante :

5' TTT CAA AAG AAA ACA CGA CAC TAT TTT ATT GCT GCA GTG
GAG AGG CTC TGG GAT TAT GGG ATG 3',

ou
b) une séquence nucléotidique qui est complémentaire de 1a séquence de la partie a).

**2.** Molécule d'acide nucléique selon la revendication 1, qui code pour la séquence d'acides aminés suivante :

PHE-GLN-LYS-LYS-THR-ARG-HIS-TYR-PHE-ILE-ALA-ALA-VAL-GLU-
ARG-LEU-TRP-ASP-TYR-GLY-MET.

**3.** Molécule d'acide nucléique selon la revendication 1 ou 2, dans laquelle l'acide nucléique est un ADN.

**4.** Molécule d'acide nucléique selon l'une quelconque des revendications précédentes, comportant la séquence nucléotidique exprimée du fragment *Eco*RI de 14,43 kb du segment d'insertion Facteur VIII:C génomique humain du bactériophage λFVIII23D (Numéro ATCC 40094).

**5.** Molécule d'acide nucléique selon l'une quelconque des revendications 1 à 3, comportant la séquence nucléotidique exprimée du fragment *Eco*RI ayant une taille d'environ 4,4 kb du segment d'insertion Facteur VIII:C génomique humain du bactériophage λFVIII23D (Numéro ATCC 40094).

**6.** Vecteur comportant une molécule d'acide nucléique selon l'une quelconque des revendications précédentes.

**7.** Vecteur selon la revendication 6, qui est un vecteur d'expression.

**8.** Vecteur selon la revendication 6, qui est un virus de mammifère recombinant.

**9.** Procédé pour générer un polypeptide comportant la séquence d'acides aminés suivante PHE-GLN-LYS-LYS-THR-ARG-HIS-TYR-PHE-ILE-ALA-ALA-VAL-GLU-ARG-LEU-TRP-ASP-TYR-GLY-MET, comportant l'introduction dans une cellule hôte d'une molécule d'acide nucléique recombinante selon l'une quelconque des revendications 1 à 5, ou d'un vecteur selon l'une quelconque des revendications 6 à 8.

**10.** Composition de cellule hôte préparée en introduisant dans une cellule hôte une molécule d'acide nucléique selon l'une quelconque des revendications 1 à 5, ou un vecteur selon l'une quelconque des revendications 6 à B et en mettant en croissance ladite cellule hôte.

**11.** Composition de cellule hôte selon la revendication 10, dans laquelle ladite cellule hôte est une cellule hôte de *E. coli* ou une cellule hôte de mammifère.

**12.** Procédé de préparation d'un vecteur d'ADN recombinant, comportant l'introduction d'une molécule d'acide nucléique selon l'une quelconque des revendications 1 à 5 dans un vecteur.

**13.** Procédé selon la revendication 12, comportant de plus l'introduction du vecteur d'acide nucléique recombinant dans une cellule hôte.

**14.** Procédé selon la revendication 13, comportant de plus la croissance de ladite cellule hôte pour fournir une composition de cellules hôtes comportant ledit vecteur recombinant.

**15.** Utilisation d'une molécule d'acide nucléique selon l'une quelconque des revendications 1 à 5, ou d'un vecteur selon l'une quelconque des revendications 6 à 8, pour la fabrication d'un médicament pour le traitement ou le diagnostic de l'hémophilie A.

**16.** Utilisation d'une molécule d'acide nucléique selon l'une quelconque des revendications 1 à 5, ou d'un vecteur selon l'une quelconque des revendications 6 à 8, pour l'expression d'une protéine Facteur VIII:C humaine de pleine longueur ou d'une partie de celle-ci.